# EUROPEAN PATENT SPECIFICATION

(11) **EP 3 347 054 B1**
(45) Date of publication and mention of the grant of the patent: **05.05.2021**
(21) Application number: 16765958.0
(22) Date of filing: 09.09.2016
(51) Int. Cl.: A61K 47/68, A61K 38/07, A61P 35/00

(54) **DOSING REGIMENS FOR ANTI-TF-ANTIBODY DRUG-CONJUGATES**
DOSIERSCHEMATA FÜR ANTI-TF-ANTIKÖRPER-ARZNEIMITTELKONJUGATE
SCHÉMA POSOLOGIQUE POUR DES CONJUGUÉS MÉDICAMENT-ANTICORPS ANTI-TF

(30) Priority: 11.09.2015 US 201562217320 P
(43) Date of publication of application: 18.07.2018
(73) Proprietor: Genmab A/S, 1560 Copenhagen V (DK)
(72) Inventor: LISBY, Steen, 3100 Hornbaek (DK); WHITING, Nancy Cherry, Seattle, Washington 98115 (US)
(74) Representative: Genmab A/S
(86) International application number: PCT/EP2016/071336
(87) International publication number: WO 2017/042352

(56) References cited:
- WO-A2-2011/157741

### SEQUENCE LISTING

<110> Genmab A/S
<120> NEW DOSING REGIMENS FOR ANTI-TF-ANTIBODY DRUG-CONJUGATES
<130> P/0094-WO
<160> 32
<170> PatentIn version 3.5
<210> 1
   <211> 118
   <212> PRT
   <213> Artificial Sequence
<220>
   <223> N/A
<400> 1
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Tyr
               20                  25                  30
   Ala Met Ser Trp Val Arg Gln Ala Pro Gly Lys Gly Leu Glu Trp Val
           35                  40                  45
   Ser Ser Ile Ser Gly Ser Gly Asp Tyr Thr Tyr Tyr Thr Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Ser Pro Trp Gly Tyr Tyr Leu Asp Ser Trp Gly Gln Gly Thr
               100                 105                 110
   Leu Val Thr Val Ser Ser
           115
<210> 2
   <211> 8
   <212> PRT
   <213> Artificial Sequence
<220>
   <223> N/A
<400> 2
   Gly Phe Thr Phe Ser Asn Tyr Ala
   1               5
<210> 3
   <211> 8
   <212> PRT
   <213> Artificial Sequence
<220>
   <223> N/A
<400> 3
   Ile Ser Gly Ser Gly Asp Tyr Thr
   1               5
<210> 4
   <211> 11
   <212> PRT
   <213> Artificial Sequence
<220>
   <223> N/A
<400> 4
   Ala Arg Ser Pro Trp Gly Tyr Tyr Leu Asp Ser
   1               5                   10
<210> 5
   <211> 118
   <212> PRT
   <213> Artificial Sequence
<220>
   <223> N/A
<400> 5
   Gln Val Gln Leu Val Gln Ser Gly Ala Glu Val Arg Lys Pro Gly Ser
   1               5                   10                  15
   Ser Val Lys Val Ser Cys Lys Ala Ser Gly Gly Ser Phe Asn Asn Tyr
               20                  25                  30
   Pro Ile Phe Trp Val Arg Gln Ala Pro Gly Gln Gly Phe Glu Trp Met
           35                  40                  45
   Gly Arg Ile Ile Pro Ile Leu Gly Ile Thr Ala Tyr Ala Gln Lys Phe
       50                  55                  60
   Gln Gly Arg Val Thr Ile Thr Ala Asp Lys Ser Thr Ser Thr Ala Tyr
   65                  70                  75                  80
   Met Glu Leu Asn Ser Leu Arg Ser Glu Asp Thr Ala Val Tyr Tyr Cys
                   85                  90                  95
   Ala Gly Gly Asp Asp Leu Asp Ala Phe Asp Ile Trp Gly Gln Gly Thr
               100                 105                 110
   Met Val Ser Val Ser Ser
           115
<210> 6
   <211> 8
   <212> PRT
   <213> Artificial Sequence
<220>
   <223> N/A
<400> 6
   Gly Gly Ser Phe Asn Asn Tyr Pro
   1               5
<210> 7
   <211> 8
   <212> PRT
   <213> Artificial Sequence
<220>
   <223> N/A
<400> 7
   Ile Ile Pro Ile Leu Gly Ile Thr
   1               5
<210> 8
   <211> 11
   <212> PRT
   <213> Artificial Sequence
<220>
   <223> N/A
<400> 8
   Ala Gly Gly Asp Asp Leu Asp Ala Phe Asp Ile
   1               5                   10
<210> 9
   <211> 120
   <212> PRT
   <213> Artificial Sequence
<220>
   <223> N/A
<400> 9
   Gln Val Gln Leu Val Glu Ser Gly Gly Gly Val Val Gln Pro Gly Arg
   1               5                   10                  15
   Ser Leu Arg Leu Ser Cys Ala Gly Ser Gly Phe Thr Phe Asn Arg Tyr
               20                  25                  30
   Ala Met Tyr Trp Val Arg Gln Ala Pro Gly Lys Gly Leu Asp Trp Val
           35                  40                  45
   Ala Val Ile Ser Asn Asp Gly Ile Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp His Thr Met Val Arg Gly Ala Phe Asp Tyr Trp Gly Gln
               100                 105                 110
   Gly Thr Leu Val Thr Val Ser Ser
           115                 120
<210> 10
   <211> 8
   <212> PRT
   <213> Artificial Sequence
<220>
   <223> N/A
<400> 10
   Gly Phe Thr Phe Asn Arg Tyr Ala
   1               5
<210> 11
   <211> 8
   <212> PRT
   <213> Artificial Sequence
<220>
   <223> N/A
<400> 11
   Ile Ser Asn Asp Gly Ile Asn Lys
   1               5
<210> 12
   <211> 13
   <212> PRT
   <213> Artificial Sequence
<220>
   <223> N/A
<400> 12
   Ala Arg Asp His Thr Met Val Arg Gly Ala Phe Asp Tyr
   1               5                   10
<210> 13
   <211> 119
   <212> PRT
   <213> Artificial Sequence
<220>
   <223> N/A
<400> 13
   Gln Val Gln Leu Val Glu Ser Gly Gly Gly Val Val Gln Pro Gly Arg
   1               5                   10                  15
   Ser Leu Arg Leu Ser Cys Val Ala Ser Gly Phe Thr Val Ser Asn Asp
               20                  25                  30
   Gly Met His Trp Val Arg Gln Ala Pro Gly Lys Gly Leu Glu Trp Val
           35                  40                  45
   Ala Leu Ile Trp Tyr Asp Gly Val Asn Lys Asn Tyr Ala Asp Ser Val
       50                  55                  60
   Lys Gly Arg Phe Thr Ile Ser Arg Asp Lys Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Arg Pro Gly Thr Phe Tyr Gly Leu Asp Val Trp Gly Gln Gly
               100                 105                 110
   Thr Thr Val Thr Val Ser Ser
           115
<210> 14
   <211> 8
   <212> PRT
   <213> Artificial Sequence
<220>
   <223> N/A
<400> 14
   Gly Phe Thr Val Ser Asn Asp Gly
   1               5
<210> 15
   <211> 8
   <212> PRT
   <213> Artificial Sequence
<220>
   <223> N/A
<400> 15
   Ile Trp Tyr Asp Gly Val Asn Lys
   1 5
<210> 16
   <211> 12
   <212> PRT
   <213> Artificial Sequence
<220>
   <223> N/A
<400> 16
   Ala Arg Arg Pro Gly Thr Phe Tyr Gly Leu Asp Val
   1 5 10
<210> 17
   <211> 107
   <212> PRT
   <213> Artificial Sequence
<220>
   <223> N/A
<400> 17
   Asp Ile Gln Met Thr Gln Ser Pro Pro Ser Leu Ser Ala Ser Ala Gly
   1 5 10 15
   Asp Arg Val Thr Ile Thr Cys Arg Ala Ser Gln Gly Ile Ser Ser Arg
   20 25 30
   Leu Ala Trp Tyr Gln Gln Lys Pro Glu Lys Ala Pro Lys Ser Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Asn Ser Tyr Pro Tyr
                   85                  90                  95
   Thr Phe Gly Gln Gly Thr Lys Leu Glu Ile Lys
               100                 105
<210> 18
   <211> 6
   <212> PRT
   <213> Artificial Sequence
<220>
   <223> N/A
<400> 18
   Gln Gly Ile Ser Ser Arg
   1               5
<210> 19
   <211> 3
   <212> PRT
   <213> Artificial Sequence
<220>
   <223> N/A
<400> 19
   Ala Ala Ser
   1
<210> 20
   <211> 9
   <212> PRT
   <213> Artificial Sequence
<220>
   <223> N/A
<400> 20
   Gln Gln Tyr Asn Ser Tyr Pro Tyr Thr
   1               5
<210> 21
   <211> 107
   <212> PRT
   <213> Artificial Sequence
<220>
   <223> N/A
<400> 21
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ser Trp
   20 25 30
   Leu Ala Trp Tyr Gln Gln Lys Pro Glu Lys Ala Pro Lys Ser Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Asn Ser Tyr Pro Tyr
   85 90 95
   Thr Phe Gly Gln Gly Thr Lys Leu Glu Ile Lys
   100 105
<210> 22
   <211> 6
   <212> PRT
   <213> Artificial Sequence
<220>
   <223> N/A
<400> 22
   Gln Gly Ile Ser Ser Trp
   1 5
<210> 23
   <211> 3
   <212> PRT
   <213> Artificial Sequence
<220>
   <223> N/A
<400> 23
   Ala Ala Ser
   1
<210> 24
   <211> 9
   <212> PRT
   <213> Artificial Sequence
<220>
   <223> N/A
<400> 24
   Gln Gln Tyr Asn Ser Tyr Pro Tyr Thr
   1 5
<210> 25
   <211> 107
   <212> PRT
   <213> Artificial Sequence
<220>
   <223> N/A
<400> 25
   Glu Ile Val Leu Thr Gln Ser Pro Ala Thr Leu Ser Leu Ser Pro Gly
   1 5 10 15
   Glu Arg Ala Thr Leu Ser Cys Arg Ala Ser Gln Ser Val Ser Ser Tyr
   20 25 30
   Leu Ala Trp Tyr Gln Gln Lys Pro Gly Gln Ala Pro Arg Leu Leu Ile
   35 40 45
   Tyr Asp Ala Ser Asn Arg Ala Thr Gly Ile Pro Ala Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Ala Val Tyr Tyr Cys Gln Gln Arg Ser Asn Trp Pro Leu
   85 90 95
   Thr Phe Gly Gly Gly Thr Lys Val Glu Ile Lys
   100 105
<210> 26
   <211> 6
   <212> PRT
   <213> Artificial Sequence
<220>
   <223> N/A
<400> 26
   Gln Ser Val Ser Ser Tyr
   1 5
<210> 27
   <211> 3
   <212> PRT
   <213> Artificial Sequence
<220>
   <223> N/A
<400> 27
   Asp Ala Ser
   1
<210> 28
   <211> 9
   <212> PRT
   <213> Artificial Sequence
<220>
   <223> N/A
<400> 28
   Gln Gln Arg Ser Asn Trp Pro Leu Thr
   1 5
<210> 29
   <211> 107
   <212> PRT
   <213> Artificial Sequence
<220>
   <223> N/A
<400> 29
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Ser Leu
   85 90 95
   Thr Phe Gly Gly Gly Thr Lys Val Glu Ile Lys
   100 105
<210> 30
   <211> 7
   <212> PRT
   <213> Artificial Sequence
<220>
   <223> N/A
<400> 30
   Gln Ser Val Ser Ser Ser Tyr
   1               5
<210> 31
   <211> 3
   <212> PRT
   <213> Artificial Sequence
<220>
   <223> N/A
<400> 31
   Gly Ala Ser
   1
<210> 32
   <211> 8
   <212> PRT
   <213> Artificial Sequence
<220>
   <223> N/A
<400> 32
   Gln Gln Tyr Gly Ser Ser Leu Thr
   1               5

## Description

### FIELD OF THE INVENTION

The present invention relates to an anti-TF antibody drug conjugate and to a pharmaceutical composition comprising the antibody drug-conjugate for use in the treatment of a solid cancer wherein the anti-TF antibody drug conjugate is administered to a subject in need thereof in cycles comprising administration once a week for three consecutive weeks followed by a one week of resting period without any administration of the anti-TF antibody drug conjugate so that each cycle time is 28 days including the resting period wherein the dose of the anti-TF antibody drug conjugate is between 0.8 mg/kg and 2.4 mg/kg of the subject's body weight.

### BACKGROUND OF THE INVENTION

Tissue factor (TF), also called thromboplastin, factor III or CD142 is a protein present in subendothelial tissue, platelets, and leukocytes necessary for the initiation of thrombin formation from the zymogen prothrombin. Thrombin formation ultimately leads to the coagulation of blood. Tissue factor enables cells to initiate the blood coagulation cascades, and it functions as the high-affinity receptor for the coagulation factor VII (FVII), a serine protease. The resulting complex provides a catalytic event that is responsible for initiation of the coagulation protease cascades by specific limited proteolysis. Unlike the other cofactors of these protease cascades, which circulate as nonfunctional precursors, this factor is a potent initiator that is fully functional when expressed on cell surfaces.

Tissue factor is the cell surface receptor for the serine protease factor VIIa (FVIIa). Binding of FVIIa to tissue factor starts signaling processes inside the cell, said signaling function playing a role in angiogenesis. Whereas angiogenesis is a normal process in growth and development, as well as in wound healing, it is also a fundamental step in the transition of tumors from a dormant state to a malignant state: when cancer cells gain the ability to produce proteins that participate in angiogenesis, so called angiogenic growth factors, these proteins are released by the tumor into nearby tissues, and stimulate new blood vessels to sprout from existing healthy blood vessels toward and into the tumor. Once new blood vessels enter the tumor, it can rapidly expand its size and invade local tissue and organs. Through the new blood vessels, cancer cells may further escape into the circulation and lodge in other organs to form new tumors (metastases).

### Solid cancers known to express tissue factor

Tissue factor (TF) is aberrantly expressed in many solid cancers including bladder, cervical, endometrial, esophageal, head and neck, lung, ovarian, pancreatic and prostate cancer. Expression has been described on tumor cells and the tumor vasculature, and has been associated with poor disease prognosis and increased metastatic properties (Forster, 2006, Clinica Chimica Acta ).

### Bladder

The most common type of bladder cancer is transitional cell carcinoma or urothelial carcinoma, which begins in urothelial cells that line the inside of the bladder. Other types of bladder cancer include squamous cell carcinoma and adenocarcinoma. 70-80% of patients with newly diagnosed bladder cancer will present with superficial or stage I bladder tumors. These patients often can be cured, but not when the cancer is muscle-invasive or metastatic. Even though most patients with advanced/metastatic disease will receive therapy, the median survival for standard gemcitabine plus cisplatin (GC) treatment is only about 14 months. Even though the GC regimen is better tolerated than the formerly standard treatment regimen methotrexate, vinblastine, doxorubicin (adriamycin) and cisplatin (MVAC), it did not improve overall survival in a phase III trial comparing the two regimens (Roberts, 2006, Annals of Oncology). Clearly there is room for improvement and need for further exploration of effective therapies in the metastatic setting.

### Cervical

The main types of cervical cancer are squamous cell carcinoma and adenocarcinoma. Long-lasting infections with human papillomavirus (HPV) type 16 and 18 cause almost all cases of cervical cancer. The standard for first-line therapy was platinum plus a taxane. Recently, bevacizumab (anti-VEGF) was approved by the US FDA for use in combination with chemotherapy, and this greatly improved overall survival in clinical trials. Even though the median overall survival exceeds one year now, the five-year relative survival for stage IV cervical cancer is only 15%, highlighting the unmet need for this disease.

### Endometrial

Endometrial cancer is the main type of uterine cancer. Most endometrial cancers are adenocarcinomas. Management of endometrial cancer relies on single agents and combinations of traditional chemotherapy that have proven efficacy in other cancers (e.g. breast and ovarian). The leading chemotherapies to treat endometrial cancer are doxorubicin or taxane with a platinum agent. The overall survival on these therapies is about one year. Clearly there is room for improvement and the need for further exploration of novel therapies in the metastatic setting.

### Esophagus

The most common types of esophageal cancer are squamous cell carcinoma and adenocarcinoma. Esophageal cancer is often diagnosed at an advanced stage, because there are no early signs or symptoms. The folinic acid (leucovorin), fluorouracil (5-FU), oxaliplatin (FOLFOX) regimen and platinum doublets are commonly used to treat esophageal cancer. These therapies offer limited efficacy, achieving median survival of approximately 11 months. Therefore, there is a great unmet need for the development of more efficacious therapies that can improve survival in the first-line setting. The prognosis becomes even worse as patients progress through later lines of therapy where no well-defined standards exist, leaving physicians to choose from a variety of regimens and most often relying on FOLFOX, folinic acid, fluorouracil and irinotecan hydrochloride (FOLFIRI), or a taxane in second-line. Monotherapy is typical in the third-line setting, with irinotecan, capecitabine and gemcitabine as the most utilized agents. However, less than one-half of patients are actually treated with a second line of therapy, and that decreases further to less than one-third of those patients receiving a third-line, highlighting the need for more treatment options across the course of the disease.

### Head & Neck

Head and neck cancers include cancers of the lip and oral cavity, pharynx (nasopharynx, oropharynx, and hypopharynx) and larynx. Most head and neck cancers begin in squamous cells. For locally advanced and metastatic head and neck cancer, systemic therapy, with or without surgery or radiotherapy, becomes the primary treatment modality. Cisplatin, alone or in combination with 5-FU is the foundation of systemic therapy. In the US, Erbitux (cetuximab) in combination with carboplatin and 5-FU is preferred for first-line, Erbitux for second-line, and Erbitux or Xeloda (capecitabine) for third-line. In Western Europe, cisplatin plus 5-FU (fluorouracil) is the preferred first-line regimen, docetaxel is preferred for second line, while Erbitux is most frequently administered to third-line patients. Erbitux was recently approved for head and neck cancer in Japan, where tegafur/gimeracil/oteracil (TS-1) is heavily utilized as a monotherapy in relapsed disease. Stage IV head and neck cancers are generally not curable. Most patients receive palliative systemic therapy or chemoradiotherapy. In some cases, chemoradiotherapy can downstage the tumor, thus making patients operable. The prognosis for metastatic/recurrent head and neck cancer is generally poor, with median survival of less than a year, thus representing an area of high unmet need where more efficacious therapies are needed. There have been subtle changes to the systemic therapy regimens utilized in the metastatic setting in the last few years. The paucity of options is reflected in the sharp reduction in patients who go on to receive third-line systemic therapy: more than one-third have perished, and more than one-quarter are unable, either from the toxicity of treatment or their advancing disease, to withstand further treatment.

### NSCLC

Lung cancer is the most common cause of cancer-related death worldwide. There are two main types of lung cancer, based on the histolology of the cells: small-cell and non-small-cell lung cancer. NSCLC is the most common type of lung cancer (85-90% of all cases). In NSCLC, tumor histology and biomarker status strongly influence which regimen a patient will receive, and the standard of care is significantly different for each patient segment. The current standard of care for squamous histology NSCLC patients (which represent approximately 20% of NSCLC) is a platinum-based chemotherapy - most often carboplatin plus either paclitaxel (CarboTaxol), Abraxane (nab-paclitaxel), or gemcitabine. For patients with non-squamous tumors (either adenocarcinoma or large cell histologies with wild type or unknown EGFR / ALK status), a platinum backbone is still standard of care, but it may be combined with Alimta (pemetrexed), Avastin (bevacizumab), or both. For patients with an EGFR or ALK mutation, a tyrosine kinase inhibitor is standard of care - Tarceva (erlotinib) and Gilotrif (Giotrif in Europe and Japan, afatinib) for patients with EGFR mutation; Xalkori (crizotinib) and Zykadia (ceritinib) for patients with ALK mutation -these patients with either EGFR/ALK mutations, typically receive upon relapse a first-line systemic therapy regimen as described above for non-squamous wild type patients, or a next generation of EGFR-targeting drug or a next generation ALK-targeting drug. Despite recent advances in therapy, lung cancer remains the leading cause of cancer death in men and women. Overall, current treatments are not considered satisfactory for most NSCLC patients, with the possible exception of very early-stage patients. In advanced-stage disease, chemotherapy offers modest improvements in median survival, although overall survival is poor. Chemotherapy has been shown to improve quality of life in these patients. With the lack of any targeted therapy approved for first-line treatment of squamous NSCLC, there is certainly a high unmet need in this population in terms of new treatment options.

### Ovarian

The most common type of ovarian cancer is ovarian epithelial cancer. The stages and treatment are the same for ovarian epithelial, fallopian tube, and primary peritoneal cancers. Platinum doublets are standard of care in first-line advanced ovarian cancer. Almost all patients with advanced disease will receive initial treatment with chemotherapy, and a median overall survival (OS) of nearly four years can be achieved in patients treated with carboplatin plus paclitaxel. Despite survival outcomes that appear better than many other advanced tumor types, in reality this disease is typically characterized by multiple relapses and numerous lines of chemotherapy. The greatest unmet need in ovarian cancer is therapy for patients who are resistant to or cannot tolerate platinum-based therapy. These patients have very few treatment options. Single-agent therapies used to treat this subset of patients include paclitaxel, pegylated liposomal doxorubicin (PLD) and topotecan. Response rate is in the 10-15% range and overall survival is approximately 12 months. In 2014, FDA approved avastin (bevacizumab) in combination with paclitaxel, PLD or topotecan as treatment for this subset of patients. The combination of avastin with chemotherapy enhanced the progression-free survival time from 3.4 months for chemotherapy alone to 6.8 months.

### Pancreatic

Most pancreatic cancers form in exocrine cells. These tumors do not secrete hormones and do not cause signs or symptoms. This makes it hard to diagnose this type of pancreatic cancer early. For most patients with exocrine pancreatic cancer, current treatments do not cure the cancer. Gemcitabine has solidly established itself as the standard on which to base treatment in chemotherapy-naive metastatic pancreatic cancer patients. The majority of newly diagnosed metastatic pancreatic cancer patients who are treated with chemotherapy receive a gemcitabine-based regimen. However, the median overall survival is only 5.9 months, one of the worst prognoses among all tumor types, highlighting the need for the development of more efficacious therapies in the first-line setting. There has been some recent success in improving overall survival with the advent of the folinic acid, 5-FU, irinotecan, oxaliplatin (FOLFIRINOX) regimen, which showed an OS of 11.1 months in first-line patients (Conroy, NEJM, 2011). However, gemcitabine is still considered standard of care.

### Prostate

Prostate cancer is the second most common cancer in men in the US, after skin cancer, and it is the second leading cause of death from cancer in men. Almost all prostate cancers are adenocarcinomas. Prostate cancer often has no early symptoms and usually grows very slowly. Most men with prostate cancer are older than 65 years and do not die from the disease. The largest unmet needs in prostate cancer are better therapies for patients with castrate-resistant prostate cancer (CRPC). CRPC is clinically defined by the level of testosterone (50ng/mL) that exists after surgical castration or chemical castration with a luteinizing hormone releasing hormone (LHRH) agonist. CRPC shows different stages: non-metastatic, first-line asymptomatic/minimally symptomatic metastatic, first-line symptomatic metastatic, second-line docetaxel-pre-treated, second-line docetaxel-naïve and third-line (e.g., patients previously treated with both docetaxel and a next-generation hormone therapy). Since survival decreases as a patient flows through these settings, the greatest unmet need in prostate cancer currently is the third-line segment as those patients have limited options.

Accordingly, there remains an unmet medical need for patients suffering from any of the above mentioned cancers and other cancers expressing TF.

It is an object of the present invention to provide methods for treating such solid cancers, known to express TF. We have previously described and characterized anti-TF-antibodies and anti-TF-ADCs in WO 2010/066803 and WO 2011/157741 respectively, which can be used in such methods, however, choosing a therapeutic dosing regimen for an antibody-drug conjugate is not straightforward, since it is difficult to predict how the balance between efficacy and safety is influenced by the dosing frequency. A weekly dosing regimen, as in the present invention, has been shown efficacious for an anti-CD30 antibody-drug conjugate (US 2011/0268751). However, since CD30 is a target that has limited expression in healthy tissue and on resting cells in non-pathological conditions and is mainly expressed on activated hematological cells and not in other organs in the body, this does not predict anything for an efficacious, yet safe, dosing regimen for a TF-targeting antibody-drug conjugate since TF is widely expressed in healthy tissue such as epithelial cells in the lung, gastrointestinal tract, cervix, bladder, breast and skin, gray matter in the brain and spinal cord, adipose tissue, mononuclear cells, cardiomyoctes, smooth muscle and glomerular tuft cells as well as on tumor tissue. In addition, TF is a target that rapidly internalizes. This may result in good efficacy, but might also induce more side effects, upon frequent dosing. Thus, it is an object of the present invention to provide a method for treating solid cancers which express TF. It is a further object of the present invention to provide a new dosing regimen for an anti-TF antibody-drug conjugate for use in a method of treating such cancers. It is a further object of the present invention to provide a new dosing regimen for an anti-TF antibody-drug conjugate which dosing regimen is more efficacious and/or safer and/or has less side-effects than the dosing regimen of dosing once every three weeks.

### SUMMARY OF THE INVENTI ON

The invention is defined by the claims. Any subject-matter falling outside the scope of the claims is provided for information purposes only. Any references in the description to methods of treatment refer to the conjugates and pharmaceutical compositions of the present invention for use in a method for treatment of the human or animal body by therapy. The present inventors have developed a new weekly dosing regimen for three consecutive weeks followed by a one week rest period of anti-TF-ADCs according to the claims, which provides a more efficacious therapeutic regimen compared to the regimen of one dose every three weeks and that it has an acceptable tolerability profile despite the frequent dosing and the ubiquitous expression in normal cells. Accordingly, the present invention relates to an anti-TF ADC for use in the treatment of solid cancers wherein the anti-TF ADC is administered to a subject in need thereof in cycles of once a week for three consecutive weeks followed by a one week rest period.

The invention further relates to a pharmaceutical composition comprising an anti-TF antibody-drug conjugate of the formula: 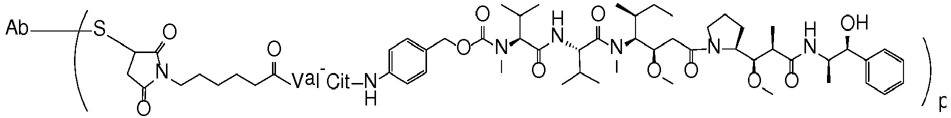 or a pharmaceutically acceptable salt thereof and a pharmaceutical acceptable carrier, wherein the mAb is an anti-TF antibody, S is a sulfur atom of the antibody, p is from 3-5, for use in a method of treating a solid cancer wherein the pharmaceutical composition is administered to a subject in need thereof in cycles of once a week for three consecutive weeks followed by a one week rest period without any administration of the anti-TF-ADC so that each cycle is 28 days including the resting period wherein the dose of the anti-TF-ADC is between 0.8 mg/kg and 2.4 mg/kg of the subject's body weight.

### DETAILED DESCRIPTION OF THE INVENTI ON

### Definitions

The terms "tissue factor", "TF", "CD142", "tissue factor antigen", "TF antigen" and "CD142 antigen" are used interchangeably herein, and, unless specified otherwise, include any variants, isoforms and species homologs of human tissue factor which are naturally expressed by cells or are expressed on cells transfected with the tissue factor gene. Tissue factor may be the sequence Genbank accession NP_001984 as used in example 1 of WO 2011/157741.

The term "immunoglobulin" refers to a class of structurally related glycoproteins consisting of two pairs of polypeptide chains, one pair of light (L) low molecular weight chains and one pair of heavy (H) chains, all four inter-connected by disulfide bonds. The structure of immunoglobulins has been well characterized. See for instance Fundamental Immunology Ch. 7 (Paul, W., ed., 2nd ed. Raven Press, N.Y. (1989)). Briefly, each heavy chain typically is comprised of a heavy chain variable region (abbreviated herein as V_{H} or VH) and a heavy chain constant region (C_{H} or CH). The heavy chain constant region typically is comprised of three domains, C_{H}1, C_{H}2, and C_{H}3. Each light chain typically is comprised of a light chain variable region (abbreviated herein as V_{L} or VL) and a light chain constant region (C_{L} or CL). The light chain constant region typically is comprised of one domain, C_{L}. The V_{H} and V_{L} regions may be further subdivided into regions of hypervariability (or hypervariable regions, which may be hypervariable in sequence and/or form of structurally defined loops), also termed complementarity-determining regions (CDRs), interspersed with regions that are more conserved, termed framework regions (FRs). Each V_{H} and V_{L} is typically composed of three CDRs and four FRs, arranged from amino-terminus to carboxy-terminus in the following order: FR1, CDR1, FR2, CDR2, FR3, CDR3, FR4 (see also Chothia and Lesk J. Mol. Biol. 196, 901-917 (1987)). Typically, the numbering of amino acid residues in this region is performed by the method described in Kabat et al., Sequences of Proteins of Immunological Interest, 5th Ed. Public Health Service, National Institutes of Health, Bethesda, MD. (1991) (phrases such as variable domain residue numbering as in Kabat or according to Kabat herein refer to this numbering system for heavy chain variable domains or light chain variable domains). Using this numbering system, the actual linear amino acid sequence of a peptide may contain fewer or additional amino acids corresponding to a shortening of, or insertion into, a FR or CDR of the variable domain. For example, a heavy chain variable domain may include a single amino acid insert (residue 52a according to Kabat) after residue 52 of V_{H} CDR2 and inserted residues (for instance residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

The term "antibody" (Ab) in the context of the present invention refers to an immunoglobulin molecule, a fragment of an immunoglobulin molecule, or a derivative of either thereof, which has the ability to specifically bind to an antigen under typical physiological conditions with a half-life of significant periods of time, such as at least about 30 minutes, at least about 45 minutes, at least about one hour, at least about two hours, at least about four hours, at least about 8 hours, at least about 12 hours, about 24 hours or more, about 48 hours or more, about 3, 4, 5, 6, 7 or more days, etc., or any other relevant functionally-defined period (such as a time sufficient to induce, promote, enhance, and/or modulate a physiological response associated with antibody binding to the antigen and/or time sufficient for the antibody to recruit an effector activity). The variable regions of the heavy and light chains of the immunoglobulin molecule contain a binding domain that interacts with an antigen. The constant regions of the antibodies (Abs) may mediate the binding of the immunoglobulin to host tissues or factors, including various cells of the immune system (such as effector cells) and components of the complement system such as C1q, the first component in the classical pathway of complement activation. As indicated above, the term antibody herein, unless otherwise stated or clearly contradicted by context, includes fragments of an antibody that retain the ability to specifically bind to the antigen. It has been shown that the antigen-binding function of an antibody may be performed by fragments of a full-length antibody. Examples of binding fragments encompassed within the term "antibody" include (i) a Fab' or Fab fragment, a monovalent fragment consisting of the V_{L}, V_{H}, C_{L} and C_{H}1 domains, or a monovalent antibody as described in WO2007059782 (Genmab A/S); (ii) F(ab')₂ fragments, bivalent fragments comprising two Fab fragments linked by a disulfide bridge at the hinge region; (iii) a Fd fragment consisting essentially of the V_{H} and C_{H}1 domains; (iv) a Fv fragment consisting essentially of the V_{L} and V_{H} domains of a single arm of an antibody, (v) a dAb fragment (Ward et al., Nature 341, 544-546 (1989)), which consists essentially of a V_{H} domain and also called domain antibodies (Holt et al; Trends Biotechnol. 2003 Nov;21(11):484-90); (vi) camelid or nanobodies (Revets et al; Expert Opin Biol Ther. 2005 Jan;5(1):111-24) and (vii) an isolated complementarity determining region (CDR). Furthermore, although the two domains of the Fv fragment, V_{L} and V_{H}, are coded for by separate genes, they may be joined, using recombinant methods, by a synthetic linker that enables them to be made as a single protein chain in which the V_{L} and V_{H} regions pair to form monovalent molecules (known as single chain antibodies or single chain Fv (scFv), see for instance Bird et al., Science 242, 423-426 (1988) and Huston et al., PNAS USA 85, 5879-5883 (1988)). Such single chain antibodies are encompassed within the term antibody unless otherwise noted or clearly indicated by context. Although such fragments are generally included within the meaning of antibody, they collectively and each independently are unique features of the present invention, exhibiting different biological properties and utility. These and other useful antibody fragments in the context of the present invention are discussed further herein. It also should be understood that the term antibody, unless specified otherwise, also includes polyclonal antibodies, monoclonal antibodies (mAbs), antibody-like polypeptides, such as chimeric antibodies and humanized antibodies, and antibody fragments retaining the ability to specifically bind to the antigen (antigen-binding fragments) provided by any known technique, such as enzymatic cleavage, peptide synthesis, and recombinant techniques. An antibody as generated can possess any isotype.

In the context of the present invention the term "ADC" refers to an antibody drug conjugate, which in the context of the present invention refers to an anti-TF antibody, which is coupled to another moiety as described in the present application.

An "anti-TF antibody" is an antibody as described above, which binds specifically to the antigen tissue factor or tissue factor antigen.

The term "human antibody", as used herein, is intended to include antibodies having variable and constant regions derived from human germline immunoglobulin sequences. The human antibodies of the invention may include amino acid residues not encoded by human germline immunoglobulin sequences (e.g., mutations introduced by random or site-specific mutagenesis in vitro or by somatic mutation in vivo). However, the term "human antibody", as used herein, is not intended to include antibodies in which CDR sequences derived from the germline of another mammalian species, such as a mouse, have been grafted onto human framework sequences.

In a preferred embodiment, the antibody of the antibody drug conjugate, or the antibody drug conjugate of the invention is isolated. An "isolated antibody" or "isolated antibody drug conjugate" as used herein, is intended to refer to an antibody or antibody drug conjugate which is substantially free of other antibodies having different antigenic specificities (for instance an isolated antibody that specifically binds to tissue factor is substantially free of antibodies that specifically bind antigens other than tissue factor). An isolated antibody drug conjugate as used herein is intended to refer to an antibody drug conjugate which is also substantially free of "free toxin", wherein "free toxin" is intended to mean toxin which is not conjugated to the antibody. The term "substantially free of" as used in relation to the toxin may in particular mean that less than 5%, such as less than 4%, or less than 3%, or less than 2%, or less than 1.5%, or less than 1%, or less than 0.5% unconjugated drug is present when determined as described in Example 16 of WO 2011/157741. An isolated antibody or isolated antibody drug conjugate that specifically binds to an epitope, isoform or variant of human tissue factor may, however, have cross-reactivity to other related antigens, for instance from other species (such as tissue factor species homologs). Moreover, an isolated antibody or antibody drug conjugate may be substantially free of other cellular material and/or chemicals. In one embodiment of the present invention, two or more "isolated" monoclonal antibodies or antibody drug conjugates having different antigen-binding specificities are combined in a well-defined composition.

When used herein in the context of two or more antibodies, the term "competes with" or "cross-competes with" indicates that the two or more antibodies compete for binding to TF, e.g. compete for TF binding in the assay as described in Example 12 of WO 10/066803. For some pairs of antibodies, competition as in the assay of Example 12 of WO 10/066803 is only observed when one antibody is coated on the plate and the other is used to compete, and not vice versa. The term "competes with" when used herein is also intended to cover such combinations of antibodies.

The terms "monoclonal antibody" or "monoclonal antibody composition" as used herein refer to a preparation of antibody molecules of single molecular composition. The monoclonal antibody or composition thereof may be drug conjugated antibodies according to the present invention. A monoclonal antibody composition displays a single binding specificity and affinity for a particular epitope. Accordingly, the term "human monoclonal antibody" refers to antibodies displaying a single binding specificity which have variable and constant regions derived from human germline immunoglobulin sequences. The human monoclonal antibodies may be generated by a hybridoma which includes a B cell obtained from a transgenic or transchromosomal non-human animal, such as a transgenic mouse, having a genome comprising a human heavy chain transgene and a light chain transgene, fused to an immortalized cell.

As used herein, the terms "binding" or "specifically binds" in the context of the binding of an antibody to a pre-determined antigen typically is a binding with an affinity corresponding to a KD of about 10-7 M or less, such as about 10-8 M or less, such as about 10-9 M or less, about 10-10 M or less, or about 10-11 M or even less when determined by for instance surface plasmon resonance (SPR) technology in a BIAcore 3000 instrument using the antigen as the ligand and the antibody as the analyte, and binds to the predetermined antigen with an affinity corresponding to a KD that is at least ten-fold lower, such as at least 100 fold lower, for instance at least 1,000 fold lower, such as at least 10,000 fold lower, for instance at least 100,000 fold lower than its affinity for binding to a non-specific antigen (e.g., BSA, casein) other than the pre-determined antigen or a closely-related antigen. The amount with which the affinity is lower is dependent on the KD of the antibody, so that when the KD of the antibody is very low (that is, the antibody is highly specific), then the amount with which the affinity for the antigen is lower than the affinity for a non-specific antigen may be at least 10,000 fold.

The term "k_{d}" (sec⁻¹), as used herein, refers to the dissociation rate constant of a particular antibody-antigen interaction. Said value is also referred to as the k_{off} value.

The term "kₐ" (M⁻¹ x sec⁻¹), as used herein, refers to the association rate constant of a particular antibody-antigen interaction.

The term "K_{D}" (M), as used herein, refers to the dissociation equilibrium constant of a particular antibody-antigen interaction.

The term "K_{A}" (M¹), as used herein, refers to the association equilibrium constant of a particular antibody-antigen interaction and is obtained by dividing the kₐ by the k_{d}.

As used herein, the term "internalization", when used in the context of a TF antibody includes any mechanism by which the antibody is internalized into a TF-expressing cell from the cell-surface. The internalization of an antibody can be evaluated in an indirect or direct assay where the effect of an internalized antibody-toxin conjugate or complex is measured (such as, e.g., the anti-kappa-ETA'assay of Example 15 of WO 2011/157741 or the internalization and cell killing assay of Example 18 of WO 2011/157741). Generally, a direct assay is used for measuring internalization of antibody drug conjugates, such as the assay described in Example 18 of WO 2011/157741, while indirect assays may be used for measuring internalization of antibodies which are then pre-incubated with a secondary conjugated antibody, such as the assay described in Example 15 of WO 2011/157741.

Antibodies may comprise functional variants of the V_{L} region, V_{H} region, or one or more CDRs of the antibodies of the examples. A functional variant of a V_{L}, V_{H}, or CDR used in the context of an anti-TF antibody still allows the antibody to retain at least a substantial proportion (at least about 50%, 60%, 70%, 80%, 90%, 95% or more) of the affinity/avidity and/or the specificity/selectivity of the parent antibody and in some cases such an anti-TF antibody may be associated with greater affinity, selectivity and/or specificity than the parent antibody.

Such functional variants typically retain significant sequence identity to the parent antibody. The percent identity between two sequences is a function of the number of identical positions shared by the sequences (i.e., % homology = # of identical positions/total # of positions x 100), taking into account the number of gaps, and the length of each gap, which need to be introduced for optimal alignment of the two sequences. The comparison of sequences and determination of percent identity between two sequences may be accomplished using a mathematical algorithm, as described in the non-limiting examples below.

The percent identity between two nucleotide sequences may be determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. The percent identity between two nucleotide or amino acid sequences may also be determined using the algorithm of E. Meyers and W. Miller, Comput. Appl. Biosci 4, 11-17 (1988)) which has been incorporated into the ALIGN program (version 2.0), using a PAM120 weight residue table, a gap length penalty of 12 and a gap penalty of 4. In addition, the percent identity between two amino acid sequences may be determined using the Needleman and Wunsch, J. Mol. Biol. 48, 444-453 (1970)) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

The sequence of CDR variants may differ from the sequence of the CDR of the parent antibody sequences through mostly conservative substitutions; for instance at least about 35%, about 50% or more, about 60% or more, about 70% or more, about 75% or more, about 80% or more, about 85% or more, about 90% or more, about 95% or more (e.g., about 65-99%, such as about 96%, 97% or 98%) of the substitutions in the variant are conservative amino acid residue replacements.

The sequence of CDR variants may differ from the sequence of the CDR of the parent antibody sequences through mostly conservative substitutions; for instance at least 10, such as at least 9, 8, 7, 6, 5, 4, 3, 2 or 1 of the substitutions in the variant are conservative amino acid residue replacements. In one embodiment the substitutions are only conservative amino acid residue substitutions. In one embodiment there are a total of no more than 3 conservative amino acid substitutions over the full parent CDR region, such as 1, 2 or 3 conservative amino acid substitutions in the VH and VL CDR regions in total.

In the context of the present invention, conservative substitutions may be defined by substitutions within the classes of amino acids reflected in one or more of the following three tables:

### Amino acid residue classes for conservative substitutions

| | |
|---|---|
| Acidic Residues | Asp (D) and Glu (E) |
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

### Alternative conservative amino acid residue substitution classes

| | | | |
|---|---|---|---|
| 1 | A | S | T |
| 2 | D | E | |
| 3 | N | Q | |
| 4 | R | K | |
| 5 | I | L | M |
| 6 | F | Y | W |

### Alternative Physical and Functional Classifications of Amino Acid Residues

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P, and T |
| Flexible residues | Q, T, K, S, G, P, D, E, and R |

More conservative substitution groupings include: valine-leucine-isoleucine, phenylalanine-tyrosine, lysine-arginine, alanine-valine, and asparagine-glutamine. Additional groups of amino acids may also be formulated using the principles described in, e.g., Creighton (1984) Proteins: Structure and Molecular Properties (2d Ed. 1993), W.H. Freeman and Company.

In one embodiment of the present invention, conservation in terms of hydropathic/hydrophilic properties and residue weight/size also is substantially retained in a variant CDR as compared to a CDR of an antibody of the examples (e.g., the weight class, hydropathic score, or both of the sequences are at least about 50%, at least about 60%, at least about 70%, at least about 75%, at least about 80%, at least about 85%, at least about 90%, at least about 95%, or more (e.g., about 65-99%) retained). For example, conservative residue substitutions may also or alternatively be based on the replacement of strong or weak based weight based conservation groups, which are known in the art.

The retention of similar residues may also or alternatively be measured by a similarity score, as determined by use of a BLAST program (e.g., BLAST 2.2.8 available through the NCBI using standard settings BLOSUM62, Open Gap=11 and Extended Gap=1). Suitable variants typically exhibit at least about 45%, such as at least about 55%, at least about 65%, at least about 75%, at least about 85%, at least about 90%, at least about 95%, or more (e.g., about 70-99%) similarity to the parent peptide.

As used herein, "isotype" refers to the immunoglobulin class (for instance IgG1, IgG2, IgG3, IgG4, IgD, IgA, IgE, or IgM) that is encoded by heavy chain constant region genes.

The term "epitope" means a protein determinant capable of specific binding to an antibody. Epitopes usually consist of surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents. The epitope may comprise amino acid residues directly involved in the binding (also called immunodominant component of the epitope) and other amino acid residues, which are not directly involved in the binding, such as amino acid residues which are effectively blocked by the specifically antigen binding peptide (in other words, the amino acid residue is within the footprint of the specifically antigen binding peptide).

As used herein, a human antibody is "derived from" a particular germline sequence if the antibody is obtained from a system using human immunoglobulin sequences, for instance by immunizing a transgenic mouse carrying human immunoglobulin genes or by screening a human immunoglobulin gene library, and wherein the selected human antibody is at least 90%, such as at least 95%, for instance at least 96%, such as at least 97%, for instance at least 98%, or such as at least 99% identical in amino acid sequence to the amino acid sequence encoded by the germline immunoglobulin gene. Typically, outside the heavy chain CDR3, a human antibody derived from a particular human germline sequence will display no more than 20 amino acid differences, e.g. no more than 10 amino acid differences, such as no more than 9, 8, 7, 6 or 5, for instance no more than 4, 3, 2, or 1 amino acid difference from the amino acid sequence encoded by the germline immunoglobulin gene.

As used herein, the term "inhibits growth" (e.g. referring to cells, such as tumor cells) is intended to include any measurable decrease in the cell growth when contacted with an anti-TF antibody drug conjugate as compared to the growth of the same cells not in contact with an anti-TF antibody drug conjugate, e.g., the inhibition of growth of a cell culture by at least about 10%, 20%, 30%, 40%, 50%, 60%, 70%, 80%, 90%, 99%, or 100%. Such a decrease in cell growth can occur by a variety of mechanisms mechanisms exerted by the anti-TF antibody and drug, either individually or in combination, e.g., antibody-dependent cell-mediated phagocytosis (ADCP), antibody-dependent cell-mediated cytotoxicity (ADCC), complement-mediated cytotoxicity (CDC), and/or apoptosis, or G2/M cell cycle arrest and apoptosis such as may be induced by an interaction of the auristatin with tubulin.

As used herein, the term "effector cell" refers to an immune cell which is involved in the effector phase of an immune response, as opposed to the cognitive and activation phases of an immune response. Exemplary immune cells include a cell of a myeloid or lymphoid origin, for instance lymphocytes (such as B cells and T cells including cytolytic T cells (CTLs)), killer cells, natural killer cells, macrophages, monocytes, eosinophils, polymorphonuclear cells, such as neutrophils, granulocytes, mast cells, and basophils. Some effector cells express specific Fc receptors (FcRs) and carry out specific immune functions. In some embodiments, an effector cell is capable of inducing ADCC, such as a natural killer cell, capable of inducing ADCC. For example, monocytes, macrophages, which express FcRs are involved in specific killing of target cells and presenting antigens to other components of the immune system, or binding to cells that present antigens. In some embodiments, an effector cell may phagocytose a target antigen or target cell. The expression of a particular FcR on an effector cell may be regulated by humoral factors such as cytokines. For example, expression of FcγRI has been found to be up-regulated by interferon γ (IFN-γ) and/or granulocyte colony stimulating factor (G-CSF). This enhanced expression increases the cytotoxic activity of FcγRI-bearing cells against target cells. An effector cell can phagocytose or lyse a target antigen or a target cell.

The term "vector", as used herein, is intended to refer to a nucleic acid molecule capable of transporting another nucleic acid to which it has been linked. One type of vector is a "plasmid", which refers to a circular double-stranded DNA loop into which additional DNA segments may be ligated. Another type of vector is a viral vector, wherein additional DNA segments may be ligated into the viral genome. Certain vectors are capable of autonomous replication in a host cell into which they are introduced (for instance bacterial vectors having a bacterial origin of replication and episomal mammalian vectors). Other vectors (such as non-episomal mammalian vectors) may be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the present invention is intended to include such other forms of expression vectors, such as viral vectors (such as replication-defective retroviruses, adenoviruses and adeno-associated viruses), which serve equivalent functions.

The term "recombinant host cell" (or simply "host cell"), as used herein, is intended to refer to a cell into which an expression vector has been introduced. It should be understood that such terms are intended to refer not only to the particular subject cell, but also to the progeny of such a cell. Because certain modifications may occur in succeeding generations due to either mutation or environmental influences, such progeny may not, in fact, be identical to the parent cell, but are still included within the scope of the term "host cell" as used herein. Recombinant host cells include, for example, transfectomas, such as CHO cells, HEK293 cells, NS/0 cells, and lymphocytic cells.

The term "transfectoma", as used herein, includes recombinant eukaryotic host cells expressing the antibody, such as CHO cells, NS/0 cells, HEK293 cells, plant cells, or fungi, including yeast cells.

The term "transgenic non-human animal" refers to a non-human animal having a genome comprising one or more human heavy and/or light chain transgenes or transchromosomes (either integrated or non-integrated into the animal's natural genomic DNA) and which is capable of expressing fully human antibodies. For example, a transgenic mouse can have a human light chain transgene and either a human heavy chain transgene or human heavy chain transchromosome, such that the mouse produces human anti-TF antibodies when immunized with TF antigen and/or cells expressing TF. The human heavy chain transgene may be integrated into the chromosomal DNA of the mouse, as is the case for transgenic mice, for instance HuMAb mice, such as HCo7, HCo17, HCo20 or HCo12 mice, or the human heavy chain transgene may be maintained extrachromosomally, as is the case for transchromosomal KM mice as described in WO02/43478. Such transgenic and transchromosomal mice (collectively referred to herein as "transgenic mice") are capable of producing multiple isotypes of human monoclonal antibodies to a given antigen (such as IgG, IgA, IgM, IgD and/or IgE) by undergoing V-D-J recombination and isotype switching. Transgenic, nonhuman animal can also be used for production of antibodies against a specific antigen by introducing genes encoding such specific antibody, for example by operatively linking the genes to a gene which is expressed in the milk of the animal.

"Treatment" refers to the administration of an effective amount of a therapeutically active compound of the present invention with the purpose of easing, ameliorating, arresting or eradicating (curing) symptoms or disease states.

An "effective amount" or "therapeutically effective amount" refers to an amount effective, at dosages and for periods of time necessary, to achieve a desired therapeutic result. A therapeutically effective amount of an anti-TF antibody drug conjugate may vary according to factors such as the disease state, age, sex, and weight of the individual, and the ability of the anti-TF antibody drug conjugate to elicit a desired response in the individual. A therapeutically effective amount is also one in which any toxic or detrimental effects of the antibody or antibody portion are outweighed by the therapeutically beneficial effects.

An "anti-idiotypic" (Id) antibody is an antibody which recognizes unique determinants generally associated with the antigen-binding site of an antibody.

The abbreviation "MMAE" refers to monomethyl auristatin E.

The abbreviations "vc" and "val-cit" refer to the dipeptide valine-citrulline.

The abbreviation "PAB" refers to the self-immolative spacer:

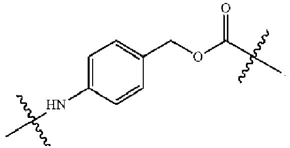

The abbreviation "MC" refers to the stretcher maleimidocaproyl:

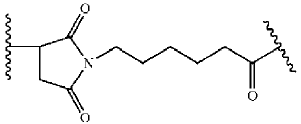

Anti-TF-MC-vc-PAB-MMAE refers to a human TF antibody conjugated to the drug MMAE through a MC-vc-PAB linker.

| **VH-region** | | **Sequence** |
|---|---|---|
| SEQ ID No: 1 | VH 011 | |
| SEQ ID No: 2 | VH 011, CDR1 | GFTFSNYA |
| SEQ ID No: 3 | VH 011, CDR2 | ISGSGDYT |
| SEQ ID No: 4 | VH 011, CDR3 | ARSPWGYYLD S |
| SEQ ID No: 5 | VH 098 | |
| SEQ ID No: 6 | VH 098, CDR1 | GGSFNNYP |
| SEQ ID No: 7 | VH 098, CDR2 | IIPILGIT |
| SEQ ID No: 8 | VH 098, CDR3 | AGGDDLDAFD I |
| SEQ ID No: 9 | VH 111 | |
| SEQ ID No: 10 | VH 111, CDR1 | GFTFNRYA |
| SEQ ID No: 11 | VH 111, CDR2 | ISNDGINK |
| SEQ ID No: 12 | VH 111, CDR3 | ARDHTMVRGA FDY |
| SEQ ID No: 13 | VH 114 | |
| SEQ ID No: 14 | VH 114, CDR1 | GFTVSNDG |
| SEQ ID No: 15 | VH 114, CDR2 | IWYDGVNK |
| SEQ ID No: 16 | VH 114, CDR3 | ARRPGTFYGL DV |

| VL-region | | |
|---|---|---|
| SEQ ID No: 17 | VL 011 | |
| SEQ ID No: 18 | VL 011, CDR1 | QGISSR |
| SEQ ID No: 19 | VL 011, CDR2 | AAS |
| SEQ ID No: 20 | VL 011, CDR3 | QQYNSYPYT |
| SEQ ID No: 21 | VL 098 | |
| SEQ ID No: 22 | VL 098, CDR1 | QGISSW |
| SEQ ID No: 23 | VL 098, CDR2 | AAS |
| SEQ ID No: 24 | VL 098, CDR3 | QQYNSYPYT |
| SEQ ID No: 25 | VL 111 | |
| SEQ ID No: 26 | VL 111, CDR1 | QSVSSY |
| SEQ ID No: 27 | VL 111, CDR2 | DAS |
| SEQ ID No: 28 | VL 111, CDR3 | QQRSNWPLT |
| SEQ ID No: 29 | VL 114 | |
| SEQ ID No: 30 | VL 114, CDR1 | QSVSSSY |
| SEQ ID No: 31 | VL 114, CDR2 | GAS |
| SEQ ID No: 32 | VL 114, CDR3 | QQYGSSLT |

Anti-TF HuMab 011 is a full length, fully human monoclonal IgG1,κ antibody comprising the VH sequence of SEQ ID No:1 and the VL sequence of SEQ ID No: 17.

Anti-TF HuMab 098 is a full length, fully human monoclonal IgG1,κ antibody comprising the VH sequence of SEQ ID No:5 and the VL sequence of SEQ ID No: 21.

Anti-TF HuMab 111 is a full length, fully human monoclonal IgG1,κ antibody comprising the VH sequence of SEQ ID No:9 and the VL sequence of SEQ ID No: 25.

Anti-TF HuMab 114 is a full length, fully human monoclonal IgG1,κ antibody comprising the VH sequence of SEQ ID No:13 and the VL sequence of SEQ ID No: 29.

Production and characterization of the antibodies and the ADCs is further described in WO 2011/157741.

### Further aspects of the invention

The present invention relates in one embodiment to an anti-TF antibody drug conjugate (anti-TF-ADC) for use according to the claims, comprising an anti-TF antibody which has been conjugated to an auristatin via a linker, said anti-TF-ADC for use in a method of treating a solid cancer wherein the anti-TF-ADC is administered to a subject in need thereof in cycles comprising administration once a week for three consecutive weeks followed by a one week resting period without any administration of anti-TF ADC so that each cycle time is 28 days including the resting period wherein the dose of the Anti-TF-ADC is between 0.8 mg/kg and 2.4 mg/kg of the subject's body weight.

The present invention relates to an anti-TF-ADC comprising an anti-TF antibody selected from the group consisting of:
(i) a VH region comprising a CDR1 region having the amino acid sequence set forth in SEQ ID NO:2, a CDR2 region having the amino acid sequence set forth in SEQ ID NO: 3, and a CDR3 region having the amino acid sequence set forth in SEQ ID NO: 4, and a VL region comprising a CDR1 region having the amino acid sequence set forth in SEQ ID NO:18, a CDR2 region having the amino acid sequence set forth in SEQ ID NO: 19, and a CDR3 region having the amino acid sequence set forth in SEQ ID NO: 20,
(ii) a VH region comprising a CDR1 region having the amino acid sequence set forth in SEQ ID NO:6, a CDR2 region having the amino acid sequence set forth in SEQ ID NO: 7, and a CDR3 region having the amino acid sequence set forth in SEQ ID NO: 8, and a VL region comprising a CDR1 region having the amino acid sequence set forth in SEQ ID NO:22, a CDR2 region having the amino acid sequence set forth in SEQ ID NO: 23, and a CDR3 region having the amino acid sequence set forth in SEQ ID NO: 24, or
(iii) a VH region comprising a CDR1 region having the amino acid sequence set forth in SEQ ID NO:10, a CDR2 region having the amino acid sequence set forth in SEQ ID NO: 11, and a CDR3 region having the amino acid sequence set forth in SEQ ID NO: 12, and a VL region comprising a CDR1 region having the amino acid sequence set forth in SEQ ID NO:26, a CDR2 region having the amino acid sequence set forth in SEQ ID NO: 27, and a CDR3 region having the amino acid sequence set forth in SEQ ID NO: 28, or
(iv) a VH region comprising a CDR1 region having the amino acid sequence set forth in SEQ ID NO:14, a CDR2 region having the amino acid sequence set forth in SEQ ID NO: 15, and a CDR3 region having the amino acid sequence set forth in SEQ ID NO: 16, and a VL region comprising a CDR1 region having the amino acid sequence set forth in SEQ ID NO: 30, a CDR2 region having the amino acid sequence set forth in SEQ ID NO: 31, and a CDR3 region having the amino acid sequence set forth in SEQ ID NO: 32, or
(v) a variant of any of said antibodies, wherein said variant has at most 1, 2 or 3 conservative amino-acid substitutions in said sequences, wherein the antibody has been conjugated to an auristatin via a linker, said anti-TF-ADC for use in a method of treating a solid cancer wherein the anti-TF-ADC is administered to a subject in need thereof once a week as a single weekly dose for three consecutive weeks followed by a one week resting period so that each cycle time is 28 days including the resting period wherein the dose of the Anti-TF-ADC is between 0.8 mg/kg and 2.4 mg/kg of the subject's body weight.

Hereby, a new dosing regimen for anti-TF ADC is provided which dosing regimen provides an efficacious therapeutic regimen and which has an acceptable tolerability profile despite the frequent dosing. In particular, this dosing regimen may be an advantage in that it is more efficacious when compared to the regimen of one dose per three weeks when used for treating the same type of solid cancers. Another advantage of this regimen may be that it has a better safety profile.

In certain embodiments of the invention the antibody of the TF-ADC comprises
(i) a VH region comprising an amino acid sequence of SEQ ID NO: 1 and a VL region comprising an amino acid sequence of SEQ ID NO: 17, or
(ii) a VH region comprising an amino acid sequence of SEQ ID NO: 5 and a VL region comprising an amino acid sequence of SEQ ID NO: 21, or
(iii) a VH region comprising an amino acid sequence of SEQ ID NO: 9 and a VL region comprising an amino acid sequence of SEQ ID NO: 25, or
(iv) a VH region comprising an amino acid sequence of SEQ ID NO: 13 and a VL region comprising an amino acid sequence of SEQ ID NO: 29. In a preferred embodiment of the invention, the anti-TF antibody of the ADC is the antibody having (i) a VH region comprising a CDR1 region having the amino acid sequence set forth in SEQ ID NO:2, a CDR2 region having the amino acid sequence set forth in SEQ ID NO: 3, and a CDR3 region having the amino acid sequence set forth in SEQ ID NO: 4, and a VL region comprising a CDR1 region having the amino acid sequence set forth in SEQ ID NO:18, a CDR2 region having the amino acid sequence set forth in SEQ ID NO: 19, and a CDR3 region having the amino acid sequence set forth in SEQ ID NO: 20.

In a preferred embodiment of the invention, the antibody is a full length antibody.

In one embodiment the antibody is a fully human monoclonal IgG1 antibody, such as an IgG1,κ.

In a further embodiment of the invention the drug conjugate of the TF-ADC is monomethyl auristatin E (MMAE): 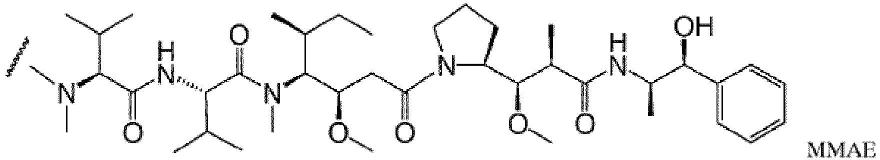 wherein the wavy line indicates the attachment site for the linker.

In an embodiment the linker of the antibody drug conjugate for use of the invention is attached to sulphydryl residues of the anti-TF antibody obtained by (partial) reduction of the anti-TF antibody.

In an embodiment of the invention the linker-auristatin of the antibody drug conjugate for use of the present invention is vcMMAE: 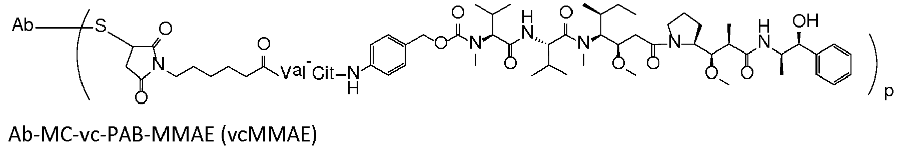 wherein p denotes a number of from 1 to 8, S represents a sulphydryl residue of the anti-TF antibody, and Ab designates the anti-TF antibody. In a preferred embodiment, p is 4 so that each anti-TF antibody molecule is conjugated with four molecules of MMAE.

Accordingly, in a preferred embodiment of the invention, the anti-TF antibody is the 011 monoclonal antibody so that the anti-TF ADC is Anti-TF HuMab 011-MC-vc-PAB-MMAE.

### Protocol

In a preferred embodiment of the present invention, the TF-ADC for use of the present invention is administered as single weekly doses for three consecutive weeks in a cycle of 28 days. In some embodiments, the dose will be administered as a single weekly dose on days 1, 8, and 15 of a 28 day treatment cycle.

Hereby, a dosing regimen is provided where the subject to be treated is dosed with a single weekly dose for three consecutive weeks followed by a resting week. This treatment schedule may also be referred to as a "weekly treatment cycle" herein and is the same as "the four-week (28 days) treatment cycle". The present invention encompasses embodiments wherein the subject remains on the four-week treatment cycle for at least 2, 3, 4, 5, 6, 7, 8, 9, 10, 11, 12 or more cycles. In another embodiment, the subject remains on this treatment cycle for between 2 and 20 cycles, such as between 2 and 15 cycles, such as between 2 and 12 cycles, such as 2 cycles, 3 cycles, 4 cycles, 5 cycles, 6 cycles, 7 cycles, 8 cycles, 9 cycles, 10 cycles, 11 cycles or 12 cycles wherein each cycle is 28 days as described above. In some embodiments, the four week treatment cycle is administered for no more than 3, no more than 4, no more than 5, or no more than 6 four-week treatment cycles.

The weekly dose of the TF-ADC for use of the invention is between 0.8 mg/kg and 2.4 mg/kg of the subject's body weight such at a dose of 0.9 mg/kg or at a dose of 1.0 mg/kg or at a dose of 1.1 mg/kg or at a dose of 1.2 mg/kg or at a dose of 1.3mg/kg or at a dose of 1.4 mg/kg or at a dose of 1.5 mg/kg or at a dose of 1.6 mg/kg or at a dose of 1.7 mg/kg or at a dose of 1.8mg/kg or at a dose of 1.9 mg/kg or at a dose of 2.0 mg/kg or at a dose of 2.1 mg/kg or at a dose of 2.2mg/kg or at a dose of 2.3 mg/kg.

In some embodiments, the weekly dose of the antibody drug conjugate will be 0.8 mg/kg body weight. In some embodiments, the weekly dose of the antibody drug conjugate will be about 0.9 mg/kg body weight. In some embodiments, the weekly dose of the antibody drug conjugate will be about 1.0 mg/kg body weight. In some embodiments, the weekly dose of the antibody drug conjugate will be about 1.1 mg/kg body weight. In some embodiments, the weekly dose of the antibody drug conjugate will be about 1.2 mg/kg body weight. In some embodiments, the weekly dose of the antibody drug conjugate will be about 1.3 mg/kg body weight. In some embodiments, the weekly dose of the antibody drug conjugate will be about 1.4 mg/kg body weight. In some embodiments, the weekly dose of the antibody drug conjugate will be about 1.5 mg/kg body weight. In some embodiments, the weekly dose of the antibody drug conjugate will be about 1.6 mg/kg body weight. In some embodiments, the weekly dose of the antibody drug conjugate will be about 1.7 mg/kg body weight. In some embodiments, the weekly dose of the antibody drug conjugate will be about 1.8 mg/kg body weight. In some embodiments, the weekly dose of the antibody drug conjugate will be about 1.9 mg/kg body weight. In some embodiments, the weekly dose of the antibody drug conjugate will be about 2.0 mg/kg body weight. In some embodiments, the weekly dose of the antibody drug conjugate will be about 2.1 mg/kg body weight. In some embodiments, the weekly dose of the antibody drug conjugate will be about 2.2 mg/kg body weight. In some embodiments, the weekly dose of the antibody drug conjugate will be about 2.3 mg/kg body weight. In some embodiments, the weekly dose of the antibody drug conjugate will be 2.4 mg/kg body weight.

In one embodiment of the invention the antibody drug conjugate is administered at a dose of about 0.9 mg/kg body weight for at least four treatment cycles of 28 days in which cycles the antibody drug conjugate is administered once a week as a single dose for three consecutive weeks followed by a resting week.

In one embodiment of the invention the antibody drug conjugate is administered at a dose of about 0.9 mg/kg body weight for at least five treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for at least six treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for at least seven treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for at least eight treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for at least nine treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for at least 10 treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for at least 11 treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for at least 12 treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for at least four treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for at least five treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for at least six treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for at least seven treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for at least eight treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for at least nine treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for at least 10 treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for at least 11 treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for at least 12 treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for at least four treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for at least five treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for at least six treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for at least seven treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for at least eight treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for at least nine treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for at least 10 treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for at least 11 treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for at least 12 treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for at least four treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for at least five treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for at least six treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for at least seven treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for at least eight treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for at least nine treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for at least 10 treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for at least 11 treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for at least 12 treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for four treatment cycles of 28 days in which cycles the antibody drug conjugate is administered once a week as a single dose for three consecutive weeks followed by a resting week.

In one embodiment of the invention the antibody drug conjugate is administered at a dose of about 0.9 mg/kg body weight for five treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for six treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for seven treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for eight treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for nine treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for 10 treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for 11 treatment cycles of 28 days in which cycles the antibody drug conjugate is administered once a week for three consecutive weeks followed by a resting week.

In one embodiment of the invention the antibody drug conjugate is administered at a dose of about 0.9 mg/kg body weight for 12 treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for four treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for five treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for six treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for seven treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for eight treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for nine treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for 10 treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for 11 treatment cycles of 28 days in which cycles the antibody drug conjugate is administered once a week for three consecutive weeks followed by a resting week.

In one embodiment of the invention the antibody drug conjugate is administered at a dose of about 1.2 mg/kg body weight for 12 treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for four treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for five treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for six treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for seven treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for eight treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for nine treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for 10 treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for 11 treatment cycles of 28 days in which cycles the antibody drug conjugate is administered once a week for three consecutive weeks followed by a resting week.

In one embodiment of the invention the antibody drug conjugate is administered at a dose of about 1.5 mg/kg body weight for 12 treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for four treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for five treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for six treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for seven treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for eight treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for nine treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for 10 treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for 11 treatment cycles of 28 days in which cycles the antibody drug conjugate is administered once a week for three consecutive weeks followed by a resting week.

In one embodiment of the invention the antibody drug conjugate is administered at a dose of about 1.8 mg/kg body weight for 12 treatment cycles of 28 days in which cycles the antibody drug conjugate is administered once a week for three consecutive weeks followed by a resting week.

In certain embodiments of the invention, the weekly dose of the TF-ADC for use of the invention is a fixed dose of between 50 mg and 200 mg such at a dose of 60 mg or a dose of 70 mg or a dose of 80 mg or a dose of 90 mg or a dose of 100 mg or a dose of 110 mg or a dose of 120 mg or a dose of 130 mg or a dose of 140 mg or a dose of 150 mg or a dose of 160 mg or a dose of 170 mg or a dose of 180 mg or a dose of 190 mg or a dose of 200 mg.

The TF-ADC is preferably administered as monotherapy. By the term "monotherapy" it is meant that the TF-ADC is the only anti-cancer agent administered to the subject during the treatment cycle. Other therapeutic agents, however, can be administered to the subject. For example, anti-inflammatory agents or other agents administered to a subject with cancer to treat symptoms associated with cancer, but not the underlying cancer itself, including, for example inflammation, pain, weight loss, and general illness can be administered during the period of monotherapy. Also, agents administered to treat potential side-effects of the TF-ADC can be administered during the period of monotherapy. A subject being treated by the present methods will preferably have completed any prior treatment with anticancer agents before administration of the TF-ADC. In some embodiments, the subject will have completed any prior treatment with anti-cancer agents at least 1 week (preferably 2, 3, 4, 5, 6, 7, or 8 weeks) prior to treatment with the TF-ADC. The subject will also, preferably, not be treated with any additional anti-cancer agents for at least 2 weeks (preferably at least 3, 4, 5, 6, 7, or 8 weeks) following completion of the first treatment cycle with the antibody drug conjugate and preferably for at least 2 weeks (preferably at least 3, 4, 5, 6, 7, or 8 weeks) following completion of the last dose of the antibody drug conjugate.

### Maintenance therapy

In some embodiment the subject will begin maintenance therapy following one or more, preferably two or more, such as following 3 or 4 or 5 or 6 or 7 or 8 or 9 or 10 or 11 or following 12 cycles of four-week treatment cycles.

In some embodiments, the subject will start maintenance therapy following an evaluation indicating that the subject has little or no detectable cancer, e.g., following an evaluation indicating that the subject has had a complete response.

As used herein, maintenance therapy refers to therapy with the antibody-drug conjugate but at a reduced administration schedule at either the same or different dosages. During maintenance therapy, the antibody-drug conjugate is preferably administered once every two weeks, once every three weeks, once every four weeks, once every five weeks, once every 6 weeks, once every 7 weeks, or once every 8 weeks. It is preferred that it is administered once every three weeks, such as on day 1 of a 21 days cycle.

Accordingly, in some embodiments, the weekly dosing cycles of three doses in 28 days can be said to be initial treatment cycles which are followed by maintenance therapy which therapy is preferably administered once every three weeks, i.e. in three weeks cycles or 21 days cycles.

In certain embodiments, the dosage of the TF-ADC administered during maintenance therapy may range e.g. from about 0.5 mg/kg body weight to about 2.4 mg/kg in cycles of 21 days as a single dose on day 1 and then again on day 22 and so on.

In one embodiment the dose of the TF-ADC for the maintenance therapy is from about 1 mg/kg body weight to about 2.4 mg/kg body weight. In another embodiment the dose of the TF-ADC for the maintenance therapy is about 1.1 mg/kg. In another embodiment the dose of the TF-ADC for the maintenance therapy is about 1.2 mg/kg. In another embodiment the dose of the TF-ADC for the maintenance therapy is about 1.3mg/kg. In another embodiment the dose of the TF-ADC for the maintenance therapy is about 1.4 mg/kg. In another embodiment the dose of the TF-ADC for the maintenance therapy is about 1.5 mg/kg. In another embodiment the dose of the TF-ADC for the maintenance therapy is about 1.6 mg/kg. In another embodiment the dose of the TF-ADC for the maintenance therapy is about 1.7 mg/kg. In another embodiment the dose of the TF-ADC for the maintenance therapy is about 1.8mg/kg. In another embodiment the dose of the TF-ADC for the maintenance therapy is about 1.9 mg/kg. In another embodiment the dose of the TF-ADC for the maintenance therapy is about 2.0 mg/kg. In another embodiment the dose of the TF-ADC for the maintenance therapy is about 2.1 mg/kg. In another embodiment the dose of the TF-ADC for the maintenance therapy is about 2.2mg/kg. In another embodiment the dose of the TF-ADC for the maintenance therapy is about 2.3 mg/kg.

In certain embodiments, the dosage of the TF-ADC administered during maintenance therapy may range e.g. from about 50 mg to about 200 mg in cycles of 21 days as a single dose on day 1 and then again on day 22 and so on.

In one embodiment the dose of the TF-ADC for the maintenance therapy is from about 50 mg to about 200 mg. In another embodiment the dose of the TF-ADC for the maintenance therapy is a dose of about 60 mg. In another embodiment the dose of the TF-ADC for the maintenance therapy is a dose of about 70 mg. In another embodiment the dose of the TF-ADC for the maintenance therapy is a dose of about 80 mg. In another embodiment the dose of the TF-ADC for the maintenance therapy is a dose of about 90 mg. In another embodiment the dose of the TF-ADC for the maintenance therapy is a dose of about 100 mg. In another embodiment the dose of the TF-ADC for the maintenance therapy is a dose of about 110 mg. In another embodiment the dose of the TF-ADC for the maintenance therapy is a dose of about 120 mg. In another embodiment the dose of the TF-ADC for the maintenance therapy is a dose of about 130 mg. In another embodiment the dose of the TF-ADC for the maintenance therapy is a dose of about 140 mg. In another embodiment the dose of the TF-ADC for the maintenance therapy is a dose of about 150 mg. In another embodiment the dose of the TF-ADC for the maintenance therapy is a dose of about 160 mg. In another embodiment the dose of the TF-ADC for the maintenance therapy is a dose of about 170 mg. In another embodiment the dose of the TF-ADC for the maintenance therapy is a dose of about 180 mg. In another embodiment the dose of the TF-ADC for the maintenance therapy is a dose of about 190 mg. In another embodiment the dose of the TF-ADC for the maintenance therapy is a dose of about 200 mg.

In certain embodiments the maintenance therapy is administered in cycles of 21 days and the number of cycles are between 2 and 20 such as between 2 and 15 cycles, such as 2 cycles, 3 cycles, 4 cycles, 5 cycles, 6 cycles, 7 cycles, 8 cycles, 9 cycles, 10 cycles, 11 cycles or 12 cycles or 13 cycles or 14 cycles or 15 cycles.

In another embodiment the maintenance therapy is administered in cycles of 21 days until partial or full remission of the cancer is detected or until an evaluation of the subject reveals that further maintenance therapy is unnecessary.

Accordingly, the present invention include embodiments wherein a subject will be administered a single weekly dose of the TF-ADC for three consecutive weeks followed by a one week resting period in four week treatment cycles for a number of cycles followed by maintenance treatment where the subject is dosed with TF-ADC once every three weeks in three weeks cycles for a number of cycles.

In one embodiment, the subjects to be treated with the present invention are subjects that have been diagnosed with a cancer that expresses Tissue Factor or subjects suspected of having such a tumor.

In one embodiment of the invention the solid cancer is head and neck cancer.

In one embodiment of the invention the solid cancer is ovary cancer.

In one embodiment of the invention the solid cancer is cervix cancer.

In one embodiment of the invention the solid cancer is endometrium cancer.

In one embodiment of the invention the solid cancer is bladder cancer

In one embodiment of the invention the solid cancer is prostate cancer, such as castration-resistant prostate cancer.

In one embodiment of the invention the solid cancer is esophagus cancer.

In one embodiment of the invention the solid cancer is lung cancer, such as non-small cell lung cancer.

In one embodiment of the invention the solid cancer is pancreatic cancer.

In other embodiments, the cancer is selected from the group consisting of tumors of the central nervous system, breast cancer, specifically triple-negative breast cancer, gastric or stomach cancer, liver and biliary cancer, pancreatic cancer, colorectal cancer, kidney cancer, malignant melanoma, sarcoma, tumors of unknown primary origin, skin cancer, glioma, cancer of the brain, uterus, and rectum.

The present invention includes TF-ADCs for use of treating a subject who is newly diagnosed and has not previously been treated for a TF-expressing cancer. The ADCs for use of the present invention can also be used to treat subjects with a refractory and/or relapsed TF-expressing cancer.

A subject with a refractory TF-expressing cancer is a subject, who has not responded to a previous anti-cancer therapy, i.e., the subject continues to experience disease progression despite therapy.

A subject with a relapsed TF-expressing cancer is a subject who has responded to a prior anti-cancer therapy for the disease at one point, but has had a re-occurrence or further progression of disease following the response.

In another aspect the invention provides a pharmaceutical composition comprising the antibody drug conjugate as defined in any of the above embodiments. In one embodiment the pharmaceutical composition further comprises a pharmaceutically acceptable carrier.

In one embodiment the present invention provides a pharmaceutical composition comprising an antibody-drug conjugate of the formula: 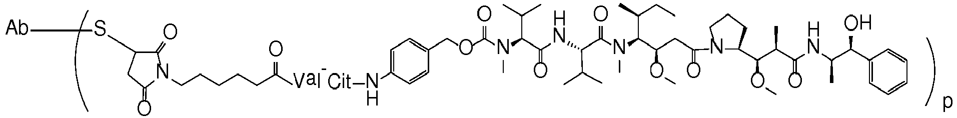 or a pharmaceutically acceptable salt thereof and a pharmaceutical acceptable carrier, wherein the mAb is an anti-TF antibody, S is a sulfur atom of the antibody, p is from 3-5, for use in a method of treating a solid cancer wherein the pharmaceutical composition is administered to a subject in need thereof as a single weekly dose of the pharmaceutical composition and the pharmaceutical composition is administered once a week for three consecutive weeks followed by a one week resting period without any administration of the anti-TF antibody drug conjugate so that each cycle time is 28 days including the resting period wherein the dose of the anti-TF ADC is between 0.8 mg/kg and 2.4 mg/kg of the subject's body weight. In a preferred embodiment p is 4.

In one embodiment the pharmaceutical composition is administered as a monotherapy.

In one embodiment the invention relates to an anti-TF antibody drug conjugate (anti-TF-ADC) for use in a method for treating a solid cancer in a subject, the method comprising administering to a subject in need thereof an anti-TF antibody drug conjugate (anti-TF-ADC) comprising an anti-TF antibody which has been conjugated to an auristatin via a linker in cycles of treatment comprising administration of anti-TF ADC once a week for three consecutive weeks followed by a one week resting period without any administration of anti-TF ADC so that each cycle time is 28 days including the resting period, according to the claims.

In another embodiment the invention relates to an anti-TF-ADC for use in a method for treating a solid cancer in a subject, the method comprising administering to a subject in need thereof in cycles comprising administration of anti-TF ADC once a week for three consecutive weeks followed by a one week resting period without any administration of anti-TF ADC so that each cycle time is 28 days including the resting period wherein the anti-TF ADC is of the formula: 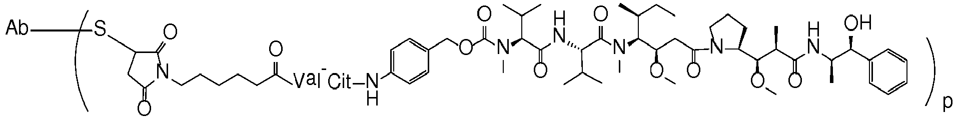 or a pharmaceutically acceptable salt thereof, wherein the mAb is an anti-TF antibody, S is a sulfur atom of the antibody, p is a number from 3-5 wherein the dose of the anti-TF-ADC is between 0.8 mg/kg and 2.4 mg/kg of the subject's body weight.

In a preferred embodiment on average p is 4.

### Outcomes

Response to therapy may include the following criteria (RECIST Criteria 1.1):

| | **Category** | **Criteria** |
|---|---|---|
| *Based on target lesions* | Complete Response (CR) | Disappearance of all target lesions. Any pathological lymph nodes must have reduction in short axis to < 10 mm. |
| | Partial Response (PR) | ≥ 30% decrease in the sum of the longest diameter (LD) of target lesions, taking as reference the baseline sum of LDs. |
| | Stable Disease (SD) | Neither sufficient shrinkage to qualify for PR nor sufficient increase to qualify for PD, taking as reference the smallest sum of LDs while in trial. |
| | Progressive Disease (PD) | ≥ 20% (and ≥ 5 mm) increase in the sum of the LDs of target lesions, taking as reference the smallest sum of the target LDs recorded while in trial or the appearance of one or more new lesions. |
| *Based on non-target lesions* | CR | Disappearance of all non-target lesions and normalization of tumor marker level. All lymph nodes must be non-pathological in size (< 10 mm short axis). |
| | SD | Persistence of one or more non-target lesion(s) or/and |
| | | maintenance of tumor marker level above the normal limits. |
| | PD | Appearance of one or more new lesions and/or unequivocal progression of existing non-target lesions. |

### Pharmaceutical composition

Upon purifying the anti-TF antibody drug conjugates they may be formulated into pharmaceutical compositions using well known pharmaceutical carriers or excipients.

The pharmaceutical compositions may be formulated with pharmaceutically acceptable carriers or diluents as well as any other known adjuvants and excipients in accordance with conventional techniques such as those disclosed in Remington: The Science and Practice of Pharmacy, 19th Edition, Gennaro, Ed., Mack Publishing Co., Easton, PA, 1995.

In another embodiment the pharmaceutical composition is formulated as disclosed in WO 2015/075201.

The pharmaceutically acceptable carriers or diluents as well as any other known adjuvants and excipients should be suitable for the antibody drug conjugate of the present invention and the chosen mode of administration. Suitability for carriers and other components of pharmaceutical compositions is determined based on the lack of significant negative impact on the desired biological properties of the chosen compound or pharmaceutical composition of the present invention (e.g., less than a substantial impact (10% or less relative inhibition, 5% or less relative inhibition, etc.)) on antigen binding.

A pharmaceutical composition of the present invention may also include diluents, fillers, salts, buffers, detergents (e. g., a nonionic detergent, such as Tween-20 or Tween-80), stabilizers (e. g., sugars or protein-free amino acids), preservatives, tissue fixatives, solubilizers, and/or other materials suitable for inclusion in a pharmaceutical composition.

Cancer cells overexpressing TF may be particularly good targets for the anti-TF antibody drug conjugates of the invention, since more antibodies may be bound per cell. Thus, in one embodiment, a cancer patient to be treated with an anti-TF antibody drug conjugate of the invention is a patient, e.g. a pancreatic cancer, lung cancer or colorectal cancer patient who has been diagnosed to have one or more mutations in K-ras and/or one or more mutations in p53 in their tumor cells. TF expression is under control of two major transforming events driving disease progression (activation of K-ras oncogene and inactivation of the p53 tumor suppressor), in a manner dependent on MEK/mitogen-activated protein kinase (MAPK) and phosphatidylinositol 3'-kinase (PI3K) (Yu et al. (2005) Blood 105:1734).

The pharmaceutical composition may be administered by any suitable route and mode. Suitable routes of administering an antibody drug conjugate of the present invention are well known in the art and may be selected by those of ordinary skill in the art.

In one embodiment, the pharmaceutical composition of the present invention is administered parenterally.

The phrases "parenteral administration" and "administered parenterally" as used herein means modes of administration other than enteral and topical administration, usually by injection, and include epidermal, intravenous, intramuscular, intraarterial, intrathecal, intracapsular, intraorbital, intracardiac, intradermal, intraperitoneal, intratendinous, transtracheal, subcutaneous, subcuticular, intraarticular, subcapsular, subarachnoid, intraspinal, intracranial, intrathoracic, epidural and intrasternal injection and infusion.

In one embodiment the pharmaceutical composition is administered by intravenous or subcutaneous injection or infusion.

Pharmaceutically acceptable carriers include any and all suitable solvents, dispersion media, coatings, antibacterial and antifungal agents, isotonicity agents, antioxidants and absorption delaying agents, and the like that are physiologically compatible with antibody drug conjugate of the present invention.

Examples of suitable aqueous-and nonaqueous carriers which may be employed in the pharmaceutical compositions of the present invention include water, saline, phosphate buffered saline, ethanol, dextrose, polyols (such as glycerol, propylene glycol, polyethylene glycol, and the like), and suitable mixtures thereof, vegetable oils, such as olive oil, corn oil, peanut oil, cottonseed oil, and sesame oil, carboxymethyl cellulose colloidal solutions, tragacanth gum and injectable organic esters, such as ethyl oleate, and/or various buffers. Other carriers are well known in the pharmaceutical arts.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the anti-TF antibody drug conjugate of the present invention, use thereof in the pharmaceutical compositions of the present invention is contemplated.

Proper fluidity may be maintained, for example, by the use of coating materials, such as lecithin, by the maintenance of the required particle size in the case of dispersions, and by the use of surfactants.

The pharmaceutical compositions of the present invention may also comprise pharmaceutically acceptable antioxidants for instance (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

The pharmaceutical compositions of the present invention may also comprise isotonicity agents, such as sugars, polyalcohols, such as mannitol, sorbitol, glycerol or sodium chloride in the compositions.

The pharmaceutical compositions of the present invention may also contain one or more adjuvants appropriate for the chosen route of administration such as preservatives, wetting agents, emulsifying agents, dispersing agents, preservatives or buffers, which may enhance the shelf life or effectiveness of the pharmaceutical composition. The anti-TF antibody drug conjugate of the present invention may be prepared with carriers that will protect the compound against rapid release, such as a controlled release formulation, including implants, transdermal patches, and microencapsulated delivery systems. Such carriers may include gelatin, glyceryl monostearate, glyceryl distearate, biodegradable, biocompatible polymers such as ethylene vinyl acetate, polyanhydrides, polyglycolic acid, collagen, polyorthoesters, and polylactic acid alone or with a wax, or other materials well known in the art. Methods for the preparation of such formulations are generally known to those skilled in the art. See e.g., Sustained and Controlled Release Drug Delivery Systems, J.R. Robinson, ed., Marcel Dekker, Inc., New York, 1978.

In one embodiment, the anti-TF antibody drug conjugate of the present invention may be formulated to ensure proper distribution in vivo. Pharmaceutically acceptable carriers for parenteral administration include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the present invention is contemplated. Supplementary active compounds may also be incorporated into the compositions.

Pharmaceutical compositions for injection must typically be sterile and stable under the conditions of manufacture and storage. The composition may be formulated as a solution, micro-emulsion, liposome, or other ordered structure suitable to high drug concentration. The carrier may be an aqueous or nonaqueous solvent or dispersion medium containing for instance water, ethanol, polyols (such as glycerol, propylene glycol, polyethylene glycol, and the like), and suitable mixtures thereof, vegetable oils, such as olive oil, and injectable organic esters, such as ethyl oleate. The proper fluidity may be maintained, for example, by the use of a coating such as lecithin, by the maintenance of the required particle size in the case of dispersion and by the use of surfactants. In many cases, it will be preferable to include isotonic agents, for example, sugars, polyalcohols such as glycerol, mannitol, sorbitol, or sodium chloride in the composition. Prolonged absorption of the injectable compositions may be brought about by including in the composition an agent that delays absorption, for example, monostearate salts and gelatin. Sterile injectable solutions may be prepared by incorporating the anti-TF antibody drug conjugate in the required amount in an appropriate solvent with one or a combination of ingredients e.g. as enumerated above, as required, followed by sterilization microfiltration. Generally, dispersions are prepared by incorporating the anti-TF antibody drug conjugate into a sterile vehicle that contains a basic dispersion medium and the required other ingredients e.g. from those enumerated above. In the case of sterile powders for the preparation of sterile injectable solutions, examples of methods of preparation are vacuum drying and freeze-drying (lyophilization) that yield a powder of the active ingredient plus any additional desired ingredient from a previously sterile-filtered solution thereof.

Sterile injectable solutions may be prepared by incorporating the anti-TF antibody drug conjugate in the required amount in an appropriate solvent with one or a combination of ingredients enumerated above, as required, followed by sterilization microfiltration. Generally, dispersions are prepared by incorporating the anti-TF antibody drug conjugate into a sterile vehicle that contains a basic dispersion medium and the required other ingredients from those enumerated above. In the case of sterile powders for the preparation of sterile injectable solutions, examples of methods of preparation are vacuum drying and freeze-drying (lyophilization) that yield a powder of the anti-TF antibody drug conjugate plus any additional desired ingredient from a previously sterile-filtered solution thereof.

## Claims

1. An anti-TF antibody drug conjugate (anti-TF-ADC) comprising an anti-TF antibody selected from the group consisting of:
(i) an anti-TF antibody having a VH region comprising a CDR1 region having the amino acid sequence set forth in SEQ ID NO:2, a CDR2 region having the amino acid sequence set forth in SEQ ID NO: 3, and a CDR3 region having the amino acid sequence set forth in SEQ ID NO: 4, and a VL region comprising a CDR1 region having the amino acid sequence set forth in SEQ ID NO:18, a CDR2 region having the amino acid sequence set forth in SEQ ID NO: 19, and a CDR3 region having the amino acid sequence set forth in SEQ ID NO: 20,
(ii) an anti-TF antibody having a VH region comprising a CDR1 region having the amino acid sequence set forth in SEQ ID NO:6, a CDR2 region having the amino acid sequence set forth in SEQ ID NO: 7, and a CDR3 region having the amino acid sequence set forth in SEQ ID NO: 8, and a VL region comprising a CDR1 region having the amino acid sequence set forth in SEQ ID NO:22, a CDR2 region having the amino acid sequence set forth in SEQ ID NO: 23, and a CDR3 region having the amino acid sequence set forth in SEQ ID NO: 24, or
(iii) an anti-TF antibody having a VH region comprising a CDR1 region having the amino acid sequence set forth in SEQ ID NO:10, a CDR2 region having the amino acid sequence set forth in SEQ ID NO: 11, and a CDR3 region having the amino acid sequence set forth in SEQ ID NO: 12, and a VL region comprising a CDR1 region having the amino acid sequence set forth in SEQ ID NO:26, a CDR2 region having the amino acid sequence set forth in SEQ ID NO: 27, and a CDR3 region having the amino acid sequence set forth in SEQ ID NO: 28, or
(iv) an anti-TF antibody having a VH region comprising a CDR1 region having the amino acid sequence set forth in SEQ ID NO:14, a CDR2 region having the amino acid sequence set forth in SEQ ID NO: 15, and a CDR3 region having the amino acid sequence set forth in SEQ ID NO: 16, and a VL region comprising a CDR1 region having the amino acid sequence set forth in SEQ ID NO: 30, a CDR2 region having the amino acid sequence set forth in SEQ ID NO: 31, and a CDR3 region having the amino acid sequence set forth in SEQ ID NO: 32, or
(v) a variant of any of said antibodies defined in (i) to (iv), wherein said variant has at most 1, 2 or 3 conservative amino-acid substitutions in the six CDR sequences,
wherein the antibody has been conjugated to an auristatin via a linker,
said anti-TF-ADC for use in a method of treating a solid cancer wherein the anti-TF-ADC is administered to a subject in need thereof in cycles comprising administration once a week for three consecutive weeks followed by a one week resting period without any administration of anti-TF ADC so that each cycle time is 28 days including the resting period wherein the dose of the anti-TF-ADC is between 0.8 mg/kg and 2.4 mg/kg of the subject's body weight.

2. An anti-TF-ADC of the formula: 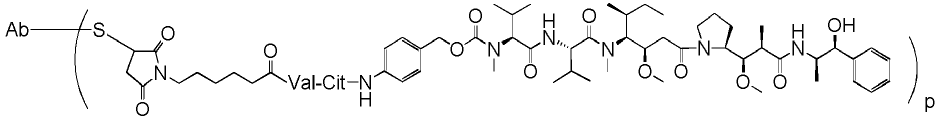 or a pharmaceutically acceptable salt thereof, wherein the mAb is an anti-TF antibody,
S is a sulfur atom of the antibody,
p is a number from 3-5,
for use in a method of treating a solid cancer wherein the anti-TF-ADC is administered to a subject in need thereof in cycles comprising administration once a week for three consecutive weeks followed by a one week resting period without any administration of anti-TF ADC so that each cycle time is 28 days including the resting period wherein the dose of the anti-TF-ADC is between 0.8 mg/kg and 2.4 mg/kg of the subject's body weight.

3. The anti-TF-ADC for use of claim 2 wherein the anti-TF antibody is selected from the group consisting of:
(i) an anti-TF antibody having a VH region comprising a CDR1 region having the amino acid sequence set forth in SEQ ID NO:2, a CDR2 region having the amino acid sequence set forth in SEQ ID NO: 3, and a CDR3 region having the amino acid sequence set forth in SEQ ID NO: 4, and a VL region comprising a CDR1 region having the amino acid sequence set forth in SEQ ID NO:18, a CDR2 region having the amino acid sequence set forth in SEQ ID NO: 19, and a CDR3 region having the amino acid sequence set forth in SEQ ID NO: 20,
(ii) an anti-TF antibody having a VH region comprising a CDR1 region having the amino acid sequence set forth in SEQ ID NO:6, a CDR2 region having the amino acid sequence set forth in SEQ ID NO: 7, and a CDR3 region having the amino acid sequence set forth in SEQ ID NO: 8, and a VL region comprising a CDR1 region having the amino acid sequence set forth in SEQ ID NO:22, a CDR2 region having the amino acid sequence set forth in SEQ ID NO: 23, and a CDR3 region having the amino acid sequence set forth in SEQ ID NO: 24, or
(iii) an anti-TF antibody having a VH region comprising a CDR1 region having the amino acid sequence set forth in SEQ ID NO:10, a CDR2 region having the amino acid sequence set forth in SEQ ID NO: 11, and a CDR3 region having the amino acid sequence set forth in SEQ ID NO: 12, and a VL region comprising a CDR1 region having the amino acid sequence set forth in SEQ ID NO:26, a CDR2 region having the amino acid sequence set forth in SEQ ID NO: 27, and a CDR3 region having the amino acid sequence set forth in SEQ ID NO: 28, or
(iv) an anti-TF antibody having a VH region comprising a CDR1 region having the amino acid sequence set forth in SEQ ID NO:14, a CDR2 region having the amino acid sequence set forth in SEQ ID NO: 15, and a CDR3 region having the amino acid sequence set forth in SEQ ID NO: 16, and a VL region comprising a CDR1 region having the amino acid sequence set forth in SEQ ID NO: 30, a CDR2 region having the amino acid sequence set forth in SEQ ID NO: 31, and a CDR3 region having the amino acid sequence set forth in SEQ ID NO: 32, or
(v) a variant of any of said antibodies defined in (i) to (iv), wherein said variant has at most 1, 2 or 3 conservative amino-acid substitutions in said six CDR sequences.

4. The anti-TF-ADC for use of any one of the preceding claims, wherein the anti-TF antibody comprises
(i) a VH region comprising an amino acid sequence of SEQ ID NO: 1 and a VL region comprising an amino acid sequence of SEQ ID NO: 17, or
(ii) a VH region comprising an amino acid sequence of SEQ ID NO: 5 and a VL region comprising an amino acid sequence of SEQ ID NO: 21, or
(iii) a VH region comprising an amino acid sequence of SEQ ID NO: 9 and a VL region comprising an amino acid sequence of SEQ ID NO: 25, or
(iv) a VH region comprising an amino acid sequence of SEQ ID NO: 13 and a VL region comprising an amino acid sequence of SEQ ID NO: 29.

5. The anti-TF-ADC for use of any one of the preceding claims wherein the anti-TF antibody is the antibody having (i) a VH region comprising a CDR1 region having the amino acid sequence set forth in SEQ ID NO:2, a CDR2 region having the amino acid sequence set forth in SEQ ID NO: 3, and a CDR3 region having the amino acid sequence set forth in SEQ ID NO: 4, and a VL region comprising a CDR1 region having the amino acid sequence set forth in SEQ ID NO:18, a CDR2 region having the amino acid sequence set forth in SEQ ID NO: 19, and a CDR3 region having the amino acid sequence set forth in SEQ ID NO: 20.

6. The anti-TF-ADC for use of any one of claims 1, 4 and 5, wherein the auristatin is monomethyl auristatin E (MMAE): 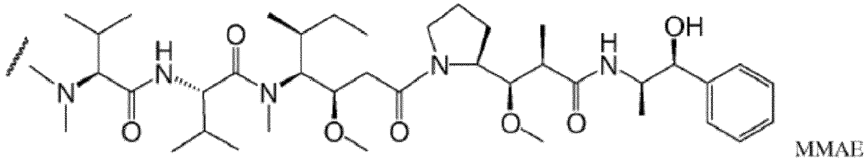 wherein the wavy line indicates the attachment site for the linker.

7. The anti-TF-ADC for use of any one of the preceding claims, wherein the linker is attached to sulphydryl residues of the anti-TF antibody obtained by (partial) reduction of the anti-TF antibody.

8. The anti-TF-ADC for use of any one of the preceding claims wherein the linker-auristatin is vcMMAE: 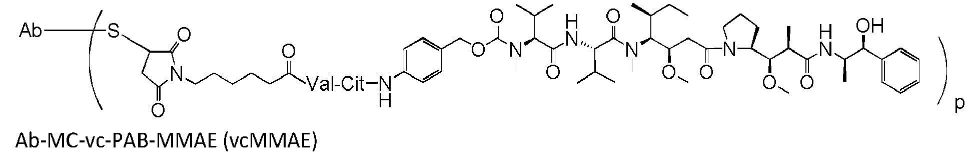 wherein p denotes a number of from 3-5, preferably 4, S represents a sulphydryl residue of the anti-TF antibody, and Ab designates the anti-TF antibody.

9. The anti-TF-ADC for use of claim 2 or 8 wherein the average p number is 4.

10. The anti-TF-ADC for use of any one of the preceding claims wherein the anti-TF-ADC is administered on days 1, 8 and 15 in the cycle of 28 days.

11. The anti-TF-ADC for use of any one of the above claims wherein the dose of anti-TF-ADC of between 0.8 mg/kg and 2.4 mg/kg of the subject's body weight, is at a dose of 0.9 mg/kg or at a dose of 1.0 mg/kg or at a dose of 1.1 mg/kg or at a dose of 1.2 mg/kg or at a dose of 1.3mg/kg or at a dose of 1.4 mg/kg or at a dose of 1.5 mg/kg or at a dose of 1.6 mg/kg or at a dose of 1.7 mg/kg or at a dose of 1.8mg/kg or at a dose of 1.9 mg/kg or at a dose of 2.0 mg/kg or at a dose of 2.1 mg/kg or at a dose of 2.2mg/kg or at a dose of 2.3 mg/kg.

12. The anti-TF-ADC for use of any one of the above claims wherein the number of cycles of 28 days is between 2 and 20, preferably the number of cycles is between 2 and 15, such as between 2 and 12, such as 2 cycles, 3 cycles, 4 cycles, 5 cycles, 6 cycles, 7 cycles, 8 cycles, 9 cycles, 10 cycles, 11 cycles or 12 cycles.

13. The anti-TF-ADC for use of any one of the above claims wherein the anti-TF-ADC is administered for at least four treatment cycles of 28 days in which cycles the anti-TF-ADC in each treatment cycle is administered once a week at a dose of 0.9 mg/kg body weight for three consecutive weeks followed by a resting week without any administration of the antibody drug conjugate.

14. The anti-TF-ADC for use of any one of the above claims wherein the anti-TF-ADC is administered at a dose of 0.9 mg/kg body weight for at least five treatment cycles of 28 days, such as 5, 6, 7, 8, 9, 10, 11 or 12 treatment cycles, in which cycles the anti-TF-ADC is administered once a week for three consecutive weeks followed by a resting week.

15. The anti-TF-ADC for use of any one of the above claims wherein the anti-TF-ADC is administered at a dose of 0.9 mg/kg body weight and wherein the solid cancer is:
a. head and neck cancer;
b. non-small cell lung cancer;
c. cervical cancer; or
d. ovarian cancer.

16. The anti-TF-ADC for use of any one of claims 1 to 12 wherein the anti-TF-ADC is administered at a dose of 1.2 mg/kg body weight and wherein the solid cancer is ovarian cancer.

17. The anti-TF-ADC for use of any one of claims 1 to 12 and claim 16 wherein the anti-TF-ADC is administered at a dose of 1.2 mg/kg body weight for at least four treatment cycles of 28 days, such as 4, 5, 6, 7, 8, 9, 10, 11 or 12 treatment cycles, in which cycles the anti-TF-ADC is administered once a week for three consecutive weeks followed by a resting week.

18. The anti-TF-ADC for use of any one of claims 1 to 12 wherein the anti-TF-ADC is administered at a dose of 1.5 mg/kg body weight for at least four treatment cycles of 28 days, such as 4, 5, 6, 7, 8, 9, 10, 11 or 12 treatment cycles, in which cycles the anti-TF-ADC is administered once a week for three consecutive weeks followed by a resting week.

19. The anti-TF-ADC for use of any one of the above claims wherein the treatment is followed by maintenance therapy.

20. The anti-TF-ADC for use of claim 19 wherein the administered dose of anti-TF-ADC for the maintenance therapy is from about 1 mg/kg body weight to about 2.4 mg/kg body weight, such as a dose of 1.1 mg/kg or at a dose of 1.2 mg/kg or at a dose of 1.3mg/kg or at a dose of 1.4 mg/kg or at a dose of 1.5 mg/kg or at a dose of 1.6 mg/kg or at a dose of 1.7 mg/kg or at a dose of 1.8mg/kg or at a dose of 1.9 mg/kg or at a dose of 2.0 mg/kg or at a dose of 2.1 mg/kg or at a dose of 2.2mg/kg or at a dose of 2.3 mg/kg.

21. The anti-TF-ADC for use of claim 19 or 20 wherein the maintenance therapy is administered in a dosing schedule of one dose per three weeks, such as on day 1 of a cycle of 21 days.

22. The anti-TF-ADC for use of claim 21 wherein the maintenance therapy is administered in cycles of 21 days and the number of cycles are between 2 and 20 such as between 2 and 15 cycles, such as 2 cycles, 3 cycles, 4 cycles, 5 cycles, 6 cycles, 7 cycles, 8 cycles, 9 cycles, 10 cycles, 11 cycles or 12 cycles or 13 cycles or 14 cycles or 15 cycles.

23. The anti-TF-ADC for use of any one of the above claims wherein the solid cancer is selected from the group comprising cancers of the pancreas, head and neck, ovary, cervix, endometrium, bladder, prostate, esophagus or lung.

24. The anti-TF-ADC for use of claim 23 wherein the prostate cancer is castration-resistant prostate cancer.

25. The anti-TF-ADC for use of claim 23 wherein the lung cancer is non-small cell lung cancer.

26. The anti-TF-ADC for use of any one of the above claims wherein the solid cancer expresses TF.

27. The anti-TF-ADC for use of any one of the above claims wherein the subject has a relapsed or refractory TF-expressing solid cancer.

28. The anti-TF-ADC for use of any one of the above claims wherein the anti-TF-ADC is administered as a monotherapy.

29. The anti-TF-ADC for use of any one of the above claims wherein the anti-TF-ADC is administered as part of a combination therapy.

30. The anti-TF-ADC for use of any one of the above claims wherein the anti-TF-ADC is comprised in a pharmaceutical composition.

31. The anti-TF-ADC for use of claim 30 wherein said pharmaceutical composition further comprises a pharmaceutically acceptable carrier.

32. The anti-TF-ADC for use of any one of the above claims wherein the anti-TF-ADC is administered as injection or infusion, preferably as intravenous infusion.

## Patentansprüche

1. Anti-TF-Antikörper-Wirkstoff-Konjugat (Anti-TF-ADC, umfassend einen Anti-TF-Antikörper, ausgewählt aus der Gruppe, bestehend aus:
(i) einem Anti-TF-Antikörper, welcher eine VH-Region besitzt, umfassend eine CDR1-Region, die die Aminosäuresequenz von SEQ ID NO:2 besitzt, eine CDR2-Region, die die Aminosäuresequenz von SEQ ID NO:3 besitzt, und eine CDR3-Region, die die Aminosäuresequenz von SEQ ID NO:4 besitzt, und eine VL-Region besitzt, umfassend eine CDR1-Region, die die Aminosäuresequenz von SEQ ID NO:18 besitzt, eine CDR2-Region, die die Aminosäuresequenz von SEQ ID NO:19 besitzt, und eine CDR3-Region, die die Aminosäuresequenz von SEQ ID NO:20 besitzt,
(ii) einem Anti-TF-Antikörper, welcher eine VH-Region besitzt, umfassend eine CDR1-Region, die die Aminosäuresequenz von SEQ ID NO:6 besitzt, eine CDR2-Region, die die Aminosäuresequenz von SEQ ID NO:7 besitzt, und eine CDR3-Region, die die Aminosäuresequenz von SEQ ID NO:8 besitzt, und eine VL-Region besitzt, umfassend eine CDR1-Region, die die Aminosäuresequenz von SEQ ID NO:22 besitzt, eine CDR2-Region, die die Aminosäuresequenz von SEQ ID NO:23 besitzt, und eine CDR3-Region, die die Aminosäuresequenz von SEQ ID NO:24 besitzt, oder
(iii) einem Anti-TF-Antikörper, welcher eine VH-Region besitzt, umfassend eine CDR1-Region, die die Aminosäuresequenz von SEQ ID NO:10 besitzt, eine CDR2-Region, die die Aminosäuresequenz von SEQ ID NO:11 besitzt, und eine CDR3-Region, die die Aminosäuresequenz von SEQ ID NO:12 besitzt, und eine VL-Region besitzt, umfassend eine CDR1-Region, die die Aminosäuresequenz von SEQ ID NO:26 besitzt, eine CDR2-Region, die die Aminosäuresequenz von SEQ ID NO:27 besitzt, und eine CDR3-Region, die die Aminosäuresequenz von SEQ ID NO:28 besitzt, oder
(iv) einem Anti-TF-Antikörper, welcher eine VH-Region besitzt, umfassend eine CDR1-Region, die die Aminosäuresequenz von SEQ ID NO:14 besitzt, eine CDR2-Region, die die Aminosäuresequenz von SEQ ID NO:15 besitzt, und eine CDR3-Region, die die Aminosäuresequenz von SEQ ID NO:16 besitzt, und eine VL-Region besitzt, umfassend eine CDR1-Region, die die Aminosäuresequenz von SEQ ID NO:30 besitzt, eine CDR2-Region, die die Aminosäuresequenz von SEQ ID NO:31 besitzt, und eine CDR3-Region, die die Aminosäuresequenz von SEQ ID NO:32 besitzt, oder
(v) einer Variante der Antikörper, wie in (i) bis (iv) definiert, wobei die Variante höchstens 1, 2 oder 3 konservative Aminosäuresubstitutionen in den sechs CDR-Sequenzen besitzt,
wobei der Antikörper über einen Linker an ein Auristatin konjugiert wurde,
wobei das Anti-TF-ADC zur Verwendung in einem Verfahren zur Behandlung von solidem Krebs ist, wobei das Anti-TF-ADC an ein Subjekt, welches es benötigt, verabreicht wird, in Zyklen, umfassend die Verabreichung einmal pro Woche für drei aufeinanderfolgende Wochen, gefolgt von einer Ruhewoche ohne jede Verabreichung des Anti-TF-ADC, so dass jede Zykluszeit 28 Tage einschließlich der Ruhephase beträgt, wobei die Dosis des Anti-TF-ADC zwischen 0,8 mg/kg und 2,4 mg/kg des Körpergewichts des Subjekts beträgt.

2. Anti-TF-ADC der Formel: 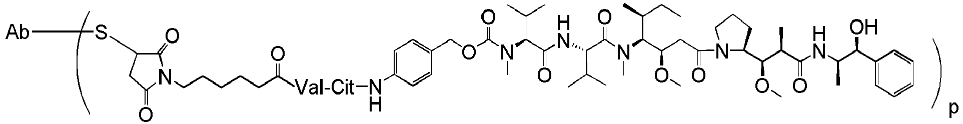 oder ein pharmazeutisch verträgliches Salz davon, wobei der mAb ein Anti-TF-Antikörper ist,
S ein Schwefelatom des Antikörpers ist,
p eine Zahl von 3-5 ist,
zur Verwendung in einem Verfahren zur Behandlung von solidem Krebs, wobei das Anti-TF-ADC an ein Subjekt, welches es benötigt, verabreicht wird, in Zyklen, umfassend die Verabreichung einmal pro Woche für drei aufeinanderfolgende Wochen, gefolgt von einer Ruhewoche ohne jede Verabreichung des Anti-TF-ADC, so dass jede Zykluszeit 28 Tage einschließlich der Ruhephase beträgt, wobei die Dosis des Anti-TF-ADC zwischen 0,8 mg/kg und 2,4 mg/kg des Körpergewichts des Subjekts beträgt.

3. Anti-TF-ADC zur Verwendung gemäß Anspruch 2, wobei der Anti-TF-Antikörper ausgewählt ist aus der Gruppe, bestehend aus:
(i) einem Anti-TF-Antikörper, welcher eine VH-Region besitzt, umfassend eine CDR1-Region, die die Aminosäuresequenz von SEQ ID NO:2 besitzt, eine CDR2-Region, die die Aminosäuresequenz von SEQ ID NO:3 besitzt, und eine CDR3-Region, die die Aminosäuresequenz von SEQ ID NO:4 besitzt, und eine VL-Region besitzt, umfassend eine CDR1-Region, die die Aminosäuresequenz von SEQ ID NO:18 besitzt, eine CDR2-Region, die die Aminosäuresequenz von SEQ ID NO:19 besitzt, und eine CDR3-Region, die die Aminosäuresequenz von SEQ ID NO:20 besitzt,
(ii) einem Anti-TF-Antikörper, welcher eine VH-Region besitzt, umfassend eine CDR1-Region, die die Aminosäuresequenz von SEQ ID NO:6 besitzt, eine CDR2-Region, die die Aminosäuresequenz von SEQ ID NO:7 besitzt, und eine CDR3-Region, die die Aminosäuresequenz von SEQ ID NO:8 besitzt, und eine VL-Region besitzt, umfassend eine CDR1-Region, die die Aminosäuresequenz von SEQ ID NO:22 besitzt, eine CDR2-Region, die die Aminosäuresequenz von SEQ ID NO:23 besitzt, und eine CDR3-Region, die die Aminosäuresequenz von SEQ ID NO:24 besitzt, oder
(iii) einem Anti-TF-Antikörper, welcher eine VH-Region besitzt, umfassend eine CDR1-Region, die die Aminosäuresequenz von SEQ ID NO:10 besitzt, eine CDR2-Region, die die Aminosäuresequenz von SEQ ID NO:11 besitzt, und eine CDR3-Region, die die Aminosäuresequenz von SEQ ID NO:12 besitzt, und eine VL-Region besitzt, umfassend eine CDR1-Region, die die Aminosäuresequenz von SEQ ID NO:26 besitzt, eine CDR2-Region, die die Aminosäuresequenz von SEQ ID NO:27 besitzt, und eine CDR3-Region, die die Aminosäuresequenz von SEQ ID NO:28 besitzt, oder
(iv) einem Anti-TF-Antikörper, welcher eine VH-Region besitzt, umfassend eine CDR1-Region, die die Aminosäuresequenz von SEQ ID NO:14 besitzt, eine CDR2-Region, die die Aminosäuresequenz von SEQ ID NO:15 besitzt, und eine CDR3-Region, die die Aminosäuresequenz von SEQ ID NO:16 besitzt, und eine VL-Region besitzt, umfassend eine CDR1-Region, die die Aminosäuresequenz von SEQ ID NO:30 besitzt, eine CDR2-Region, die die Aminosäuresequenz von SEQ ID NO:31 besitzt, und eine CDR3-Region, die die Aminosäuresequenz von SEQ ID NO:32 besitzt, oder
(v) einer Variante der Antikörper, wie in (i) bis (iv) definiert, wobei die Variante höchstens 1, 2 oder 3 konservative Aminosäuresubstitutionen in den sechs CDR-Sequenzen besitzt.

4. Anti-TF-ADC zur Verwendung gemäß einem der vorherigen Ansprüche, wobei der Anti-TF-Antikörper umfasst:
(i) eine VH-Region, umfassend eine Aminosäuresequenz von SEQ ID NO:1, und eine VL-Region, umfassend eine Aminosäuresequenz von SEQ ID NO:17, oder
(ii) eine VH-Region, umfassend eine Aminosäuresequenz von SEQ ID NO:5, und eine VL-Region, umfassend eine Aminosäuresequenz von SEQ ID NO:21, oder
(iii) eine VH-Region, umfassend eine Aminosäuresequenz von SEQ ID NO:9, und eine VL-Region, umfassend eine Aminosäuresequenz von SEQ ID NO:25, oder
(iv) eine VH-Region, umfassend eine Aminosäuresequenz von SEQ ID NO:13, und eine VL-Region, umfassend eine Aminosäuresequenz von SEQ ID NO:29.

5. Anti-TF-ADC zur Verwendung gemäß einem der vorherigen Ansprüche, wobei der Anti-TF-Antikörper der Antikörper ist, der (i) eine VH-Region besitzt, umfassend eine CDR1-Region, die die Aminosäuresequenz von SEQ ID NO:2 besitzt, eine CDR2-Region, die die Aminosäuresequenz von SEQ ID NO:3 besitzt, und eine CDR3-Region, die die Aminosäuresequenz von SEQ ID NO:4 besitzt, und eine VL-Region besitzt, umfassend eine CDR1-Region, die die Aminosäuresequenz von SEQ ID NO:18 besitzt, eine CDR2-Region, die die Aminosäuresequenz von SEQ ID NO:19 besitzt, und eine CDR3-Region, die die Aminosäuresequenz von SEQ ID NO:20 besitzt.

6. Anti-TF-ADC zur Verwendung gemäß einem der Ansprüche 1, 4 und 5, wobei das Auristatin Monomethyl-Auristatin E (MMAE) ist: 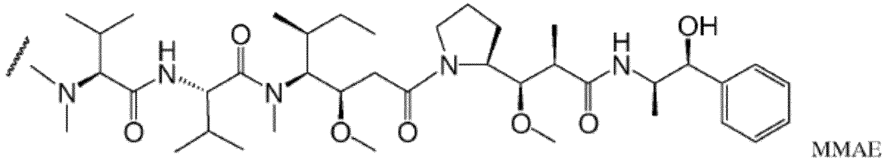 wobei die gewellte Linie die Bindestelle für den Linker kennzeichnet.

7. Anti-TF-ADC zur Verwendung gemäß einem der vorherigen Ansprüche, wobei der Linker an Sulfhydrylreste des Anti-TF-Antikörpers, welche über eine (teilweise) Reduktion des Anti-TF-Antikörpers erhalten wurden, befestigt ist.

8. Anti-TF-ADC zur Verwendung gemäß einem der vorherigen Ansprüche, wobei das Linker-Auristatin vcMMAE ist: 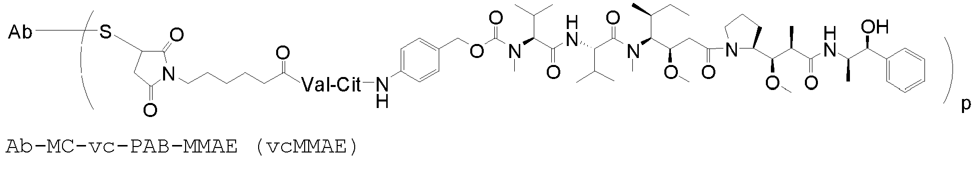 wobei p eine Zahl von 3-5 bezeichnet, vorzugsweise 4, S einen Sulfhydrylrest des Anti-TF-Antikörpers repräsentiert und Ab den Anti-TF-Antikörper bezeichnet.

9. Anti-TF-ADC zur Verwendung gemäß Anspruch 2 oder 8, wobei die durchschnittliche Anzahl von p 4 ist.

10. Anti-TF-ADC zur Verwendung gemäß einem der vorherigen Ansprüche, wobei das Anti-TF-ADC an den Tagen 1, 8 und 15 des 28-Tage-Zyklus verabreicht wird.

11. Anti-TF-ADC zur Verwendung gemäß einem der vorherigen Ansprüche, wobei die Dosis des Anti-TF-ADC zwischen 0,8 mg/kg und 2,4 mg/kg des Körpergewichts des Subjekts bei einer Dosis von 0,9 mg/kg oder einer Dosis von 1,0 mg/kg oder einer Dosis von 1,1 mg/kg oder einer Dosis von 1,2 mg/kg oder einer Dosis von 1,3 mg/kg oder einer Dosis von 1,4 mg/kg oder einer Dosis von 1,5 mg/kg oder einer Dosis von 1,6 mg/kg oder einer Dosis von 1,7 mg/kg oder einer Dosis von 1,8 mg/kg oder einer Dosis von 1,9 mg/kg oder einer Dosis von 2,0 mg/kg oder einer Dosis von 2,1 mg/kg oder einer Dosis von 2,2 mg/kg oder einer Dosis von 2,3 mg/kg ist.

12. Anti-TF-ADC zur Verwendung gemäß einem der vorherigen Ansprüche, wobei die Anzahl der 28 Tage-Zyklen 2 bis 20 beträgt, vorzugsweise die Anzahl der Zyklen 2 bis 15 beträgt, wie 2 bis 12, wie zum Beispiel 2 Zyklen, 3 Zyklen, 4 Zyklen, 5 Zyklen, 6 Zyklen, 7 Zyklen, 8 Zyklen, 9 Zyklen, 10 Zyklen, 11 Zyklen oder 12 Zyklen.

13. Anti-TF-ADC zur Verwendung gemäß einem der vorherigen Ansprüche, wobei das Anti-TF-ADC verabreicht wird für mindestens vier Behandlungszyklen von 28 Tagen, in welchen das Anti-TF-ADC in jedem Behandlungszyklus einmal pro Woche bei einer Dosis von 0,9 mg/kg Körpergewicht für drei aufeinanderfolgende Wochen, gefolgt von einer Ruhewoche ohne jede Verabreichung des Antikörper-Wirkstoff-Konjugats, verabreicht wird.

14. Anti-TF-ADC zur Verwendung gemäß einem der vorherigen Ansprüche, wobei das Anti-TF-ADC verabreicht wird bei einer Dosis von 0,9 mg/kg Körpergewicht für mindestens fünf Behandlungszyklen von 28 Tagen, wie 5, 6, 7, 8, 9, 10, 11 oder 12 Behandlungszyklen, in welchen das Anti-TF-ADC einmal pro Woche für drei aufeinanderfolgende Wochen, gefolgt von einer Ruhewoche, verabreicht wird.

15. Anti-TF-ADC zur Verwendung gemäß einem der vorherigen Ansprüche, wobei das Anti-TF-ADC mit einer Dosis von 0,9 mg/kg Körpergewicht verabreicht wird und wobei der solide Krebs:
a. Kopf- und Halskrebs;
b. nicht-kleinzelliges Lungenkarzinom;
c. Gebärmutterhalskrebs oder
d. Ovarialkarzinom ist.

16. Anti-TF-ADC zur Verwendung gemäß einem der Ansprüche 1 bis 12, wobei das Anti-TF-ADC verabreicht wird mit einer Dosis von 1,2 mg/kg Körpergewicht und wobei der solide Krebs ein Ovarialkarzinom ist.

17. Anti-TF-ADC zur Verwendung gemäß einem der Ansprüche 1 bis 12 und Anspruch 16, wobei das Anti-TF-ADC verabreicht wird bei einer Dosis von 1,2 mg/kg Körpergewicht für mindestens vier Behandlungszyklen von 28 Tagen, wie 4, 5, 6, 7, 8, 9, 10, 11 oder 12 Behandlungszyklen, in welchen das Anti-TF-ADC einmal pro Woche für drei aufeinanderfolgende Wochen, gefolgt von einer Ruhewoche, verabreicht wird.

18. Anti-TF-ADC zur Verwendung gemäß einem der Ansprüche 1 bis 12, wobei das Anti-TF-ADC verabreicht wird mit einer Dosis von 1,5 mg/kg Körpergewicht für mindestens vier Behandlungszyklen von 28 Tagen, wie 4, 5, 6, 7, 8, 9, 10, 11 oder 12 Behandlungszyklen, in welchen das Anti-TF-ADC einmal pro Woche für drei aufeinanderfolgende Wochen, gefolgt von einer Ruhewoche, verabreicht wird.

19. Anti-TF-ADC zur Verwendung gemäß einem der vorherigen Ansprüche, wobei auf die Behandlung eine Erhaltungstherapie folgt.

20. Anti-TF-ADC zur Verwendung gemäß Anspruch 19, wobei die verabreichte Dosis des Anti-TF-ADC für die Erhaltungstherapie ungefähr 1 mg/kg Körpergewicht bis ungefähr 2,4 mg/kg Körpergewicht beträgt, beispielsweise eine Dosis von 1,1 mg/kg oder eine Dosis von 1,2 mg/kg oder eine Dosis von 1,3 mg/kg oder eine Dosis von 1,4 mg/kg oder eine Dosis von 1,5 mg/kg oder eine Dosis von 1,6 mg/kg oder eine Dosis von 1,7 mg/kg oder eine Dosis von 1,8 mg/kg oder eine Dosis von 1,9 mg/kg oder eine Dosis von 2,0 mg/kg oder eine Dosis von 2,1 mg/kg oder eine Dosis von 2,2 mg/kg oder eine Dosis von 2,3 mg/kg.

21. Anti-TF-ADC zur Verwendung gemäß Anspruch 19 oder 20, wobei die Erhaltungstherapie verabreicht wird in einem Dosierungsplan von einer Dosis pro drei Wochen, zum Beispiel an Tag 1 eines 21 Tage-Zyklus.

22. Anti-TF-ADC zur Verwendung gemäß Anspruch 21, wobei die Erhaltungstherapie in 21 Tage-Zyklen verabreicht wird und die Anzahl der Zyklen 2 bis 20 beträgt, wie 2 bis 15 Zyklen, wie zum Beispiel 2 Zyklen, 3 Zyklen, 4 Zyklen, 5 Zyklen, 6 Zyklen, 7 Zyklen, 8 Zyklen, 9 Zyklen, 10 Zyklen, 11 Zyklen oder 12 Zyklen oder 13 Zyklen oder 14 Zyklen oder 15 Zyklen.

23. Anti-TF-ADC zur Verwendung gemäß einem der vorherigen Ansprüche, wobei der solide Krebs ausgewählt ist aus der Gruppe, umfassend Pankreaskrebs, Kopf- und Halskrebs, Ovarialkarzinom, Gebärmutterhalskrebs, Endometriumkarzinom, Blasenkrebs, Prostatakrebs, Speiseröhrenkrebs oder Lungenkrebs.

24. Anti-TF-ADC zur Verwendung gemäß Anspruch 23, wobei der Prostatakrebs ein kastrationsresistenter Prostatakrebs ist.

25. Anti-TF-ADC zur Verwendung gemäß Anspruch 23, wobei der Lungenkrebs nicht-kleinzelliges Lungenkarzinom ist.

26. Anti-TF-ADC zur Verwendung gemäß einem der vorherigen Ansprüche, wobei der solide Krebs TF exprimiert.

27. Anti-TF-ADC zur Verwendung gemäß einem der vorherigen Ansprüche, wobei das Subjekt einen rezidivierten oder refraktären TF-exprimierenden soliden Krebs hat.

28. Anti-TF-ADC zur Verwendung gemäß einem der vorherigen Ansprüche, wobei das Anti-TF-ADC als Monotherapie verabreicht wird.

29. Anti-TF-ADC zur Verwendung gemäß einem der vorherigen Ansprüche, wobei das Anti-TF-ADC als Teil einer Kombinationstherapie verabreicht wird.

30. Anti-TF-ADC zur Verwendung gemäß einem der vorherigen Ansprüche, wobei das Anti-TF-ADC in einer pharmazeutischen Zusammensetzung enthalten ist.

31. Anti-TF-ADC zur Verwendung gemäß Anspruch 30, wobei die pharmazeutische Zusammensetzung zusätzlich einen pharmazeutisch verträglichen Trägerstoff umfasst.

32. Anti-TF-ADC zur Verwendung gemäß einem der vorherigen Ansprüche, wobei das Anti-TF-ADC als Injektion oder Infusion, vorzugsweise als intravenöse Infusion, verabreicht wird.

## Revendications

1. Conjugué d'anticorps anti-FT et de médicament (« anti-FT-AMC »), comprenant un anticorps anti-FT choisi dans l'ensemble formé par les suivants :
i) un anticorps anti-FT comportant une région variable de chaîne lourde qui comprend une région CDR1 dont la séquence d'acides aminés est indiquée par la Séquence N° 2, une région CDR2 dont la séquence d'acides aminés est indiquée par la Séquence N° 3 et une région CDR3 dont la séquence d'acides aminés est indiquée par la Séquence N° 4, et une région variable de chaîne légère qui comprend une région CDR1 dont la séquence d'acides aminés est indiquée par la Séquence N° 18, une région CDR2 dont la séquence d'acides aminés est indiquée par la Séquence N° 19 et une région CDR3 dont la séquence d'acides aminés est indiquée par la Séquence N° 20,
ii) un anticorps anti-FT comportant une région variable de chaîne lourde qui comprend une région CDR1 dont la séquence d'acides aminés est indiquée par la Séquence N° 6, une région CDR2 dont la séquence d'acides aminés est indiquée par la Séquence N° 7 et une région CDR3 dont la séquence d'acides aminés est indiquée par la Séquence N° 8, et une région variable de chaîne légère qui comprend une région CDR1 dont la séquence d'acides aminés est indiquée par la Séquence N° 22, une région CDR2 dont la séquence d'acides aminés est indiquée par la Séquence N° 23 et une région CDR3 dont la séquence d'acides aminés est indiquée par la Séquence N° 24,
iii) un anticorps anti-FT comportant une région variable de chaîne lourde qui comprend une région CDR1 dont la séquence d'acides aminés est indiquée par la Séquence N° 10, une région CDR2 dont la séquence d'acides aminés est indiquée par la Séquence N° 11 et une région CDR3 dont la séquence d'acides aminés est indiquée par la Séquence N° 12, et une région variable de chaîne légère qui comprend une région CDR1 dont la séquence d'acides aminés est indiquée par la Séquence N° 26, une région CDR2 dont la séquence d'acides aminés est indiquée par la Séquence N° 27 et une région CDR3 dont la séquence d'acides aminés est indiquée par la Séquence N° 28,
iv) un anticorps anti-FT comportant une région variable de chaîne lourde qui comprend une région CDR1 dont la séquence d'acides aminés est indiquée par la Séquence N° 14, une région CDR2 dont la séquence d'acides aminés est indiquée par la Séquence N° 15 et une région CDR3 dont la séquence d'acides aminés est indiquée par la Séquence N° 16, et une région variable de chaîne légère qui comprend une région CDR1 dont la séquence d'acides aminés est indiquée par la Séquence N° 30, une région CDR2 dont la séquence d'acides aminés est indiquée par la Séquence N° 31 et une région CDR3 dont la séquence d'acides aminés est indiquée par la Séquence N° 32,
v) et un variant de l'un de ces anticorps définis dans les items (i) à (iv), lequel variant présente au plus 1, 2 ou 3 substitution(s) d'acide aminé conservatrice(s) dans les six séquences de région CDR,
dans lequel l'anticorps est conjugué à une auristatine par l'intermé-diaire d'un raccord,
lequel conjugué anti-FT-AMC est destiné à être utilisé dans un procédé de traitement d'un cancer solide, dans lequel le conjugué anti-FT-AMC est administré à un sujet qui en a besoin en cycles comportant une admi-nistration une fois par semaine pendant trois semaines consécutives, suivies d'un temps de relâche d'une semaine sans administration de conjugué anti-FT-AMC, de sorte que la durée de chaque cycle est de 28 jours, y compris le temps de relâche, et la dose de conjugué anti-FT-AMC vaut de 0,8 à 2,4 mg par kilogramme de poids corporel du sujet.

2. Conjugué anti-FT-AMC de formule 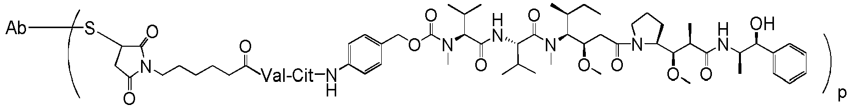 ou sel pharmacologiquement admissible d'un tel conjugué,
dans laquelle formule
- mAb représente un anticorps anti-FT,
- S représente un atome de soufre de l'anticorps,
- et l'indice p est un nombre valant de 3 à 5,
pour utilisation dans un procédé de traitement d'un cancer solide, dans lequel le conjugué anti-FT-AMC est administré à un sujet qui en a besoin en cycles comportant une administration une fois par semaine pendant trois semaines consécutives, suivies d'un temps de relâche d'une semaine sans administration de conjugué anti-FT-AMC, de sorte que la durée de chaque cycle est de 28 jours, y compris le temps de relâche, et la dose de conjugué anti-FT-AMC vaut de 0,8 à 2,4 mg par kilogramme de poids corporel du sujet.

3. Conjugué anti-FT-AMC pour utilisation conforme à la reven-dication 2, dans lequel l'anticorps anti-FT est choisi dans l'ensemble formé par les suivants :
i) un anticorps anti-FT comportant une région variable de chaîne lourde qui comprend une région CDR1 dont la séquence d'acides aminés est indiquée par la Séquence N° 2, une région CDR2 dont la séquence d'acides aminés est indiquée par la Séquence N° 3 et une région CDR3 dont la séquence d'acides aminés est indiquée par la Séquence N° 4, et une région variable de chaîne légère qui comprend une région CDR1 dont la séquence d'acides aminés est indiquée par la Séquence N° 18, une région CDR2 dont la séquence d'acides aminés est indiquée par la Séquence N° 19 et une région CDR3 dont la séquence d'acides aminés est indiquée par la Séquence N° 20,
ii) un anticorps anti-FT comportant une région variable de chaîne lourde qui comprend une région CDR1 dont la séquence d'acides aminés est indiquée par la Séquence N° 6, une région CDR2 dont la séquence d'acides aminés est indiquée par la Séquence N° 7 et une région CDR3 dont la séquence d'acides aminés est indiquée par la Séquence N° 8, et une région variable de chaîne légère qui comprend une région CDR1 dont la séquence d'acides aminés est indiquée par la Séquence N° 22, une région CDR2 dont la séquence d'acides aminés est indiquée par la Séquence N° 23 et une région CDR3 dont la séquence d'acides aminés est indiquée par la Séquence N° 24,
iii) un anticorps anti-FT comportant une région variable de chaîne lourde qui comprend une région CDR1 dont la séquence d'acides aminés est indiquée par la Séquence N° 10, une région CDR2 dont la séquence d'acides aminés est indiquée par la Séquence N° 11 et une région CDR3 dont la séquence d'acides aminés est indiquée par la Séquence N° 12, et une région variable de chaîne légère qui comprend une région CDR1 dont la séquence d'acides aminés est indiquée par la Séquence N° 26, une région CDR2 dont la séquence d'acides aminés est indiquée par la Séquence N° 27 et une région CDR3 dont la séquence d'acides aminés est indiquée par la Séquence N° 28,
iv) un anticorps anti-FT comportant une région variable de chaîne lourde qui comprend une région CDR1 dont la séquence d'acides aminés est indiquée par la Séquence N° 14, une région CDR2 dont la séquence d'acides aminés est indiquée par la Séquence N° 15 et une région CDR3 dont la séquence d'acides aminés est indiquée par la Séquence N° 16, et une région variable de chaîne légère qui comprend une région CDR1 dont la séquence d'acides aminés est indiquée par la Séquence N° 30, une région CDR2 dont la séquence d'acides aminés est indiquée par la Séquence N° 31 et une région CDR3 dont la séquence d'acides aminés est indiquée par la Séquence N° 32,
v) et un variant de l'un de ces anticorps définis dans les items (i) à (iv), lequel variant présente au plus 1, 2 ou 3 substitution(s) d'acide aminé conservatrice(s) dans les six séquences de région CDR.

4. Conjugué anti-FT-AMC pour utilisation conforme à l'une des revendications précédentes, dans lequel l'anticorps anti-FT comporte :
i) une région variable de chaîne lourde qui comprend une séquence d'acides aminés indiquée par la Séquence N° 1, et une région variable de chaîne légère qui comprend une séquence d'acides aminés indiquée par la Séquence N° 17,
ii) une région variable de chaîne lourde qui comprend une séquence d'acides aminés indiquée par la Séquence N° 5, et une région variable de chaîne légère qui comprend une séquence d'acides aminés indiquée par la Séquence N° 21,
iii) une région variable de chaîne lourde qui comprend une séquence d'acides aminés indiquée par la Séquence N° 9, et une région variable de chaîne légère qui comprend une séquence d'acides aminés indiquée par la Séquence N° 25,
iv) une région variable de chaîne lourde qui comprend une séquence d'acides aminés indiquée par la Séquence N° 13, et une région variable de chaîne légère qui comprend une séquence d'acides aminés indiquée par la Séquence N° 29.

5. Conjugué anti-FT-AMC pour utilisation conforme à l'une des revendications précédentes, dans lequel l'anticorps anti-FT est l'anti-corps (i) qui comporte une région variable de chaîne lourde comprenant une région CDR1 dont la séquence d'acides aminés est indiquée par la Séquence N° 2, une région CDR2 dont la séquence d'acides aminés est indiquée par la Séquence N° 3 et une région CDR3 dont la séquence d'acides aminés est indiquée par la Séquence N° 4, et une région varia-ble de chaîne légère comprenant une région CDR1 dont la séquence d'acides aminés est indiquée par la Séquence N° 18, une région CDR2 dont la séquence d'acides aminés est indiquée par la Séquence N° 19 et une région CDR3 dont la séquence d'acides aminés est indiquée par la Séquence N° 20.

6. Conjugué anti-FT-AMC pour utilisation conforme à l'une des revendications 1, 4 et 5, dans lequel l'auristatine est la monométhyl-auristatine E (MMAE) : 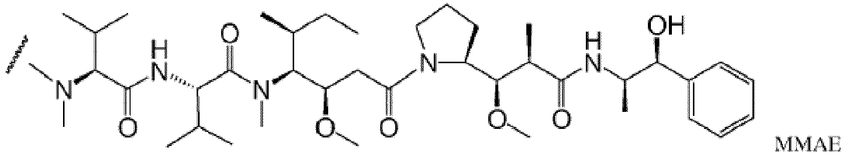 étant entendu que le trait ondulé indique le site où s'attache le raccord.

7. Conjugué anti-FT-AMC pour utilisation conforme à l'une des revendications précédentes, dans lequel le raccord est attaché à des résidus de type sulfhydryle de l'anticorps anti-FT, obtenus par réduc-tion (partielle) de l'anticorps anti-FT.

8. Conjugué anti-FT-AMC pour utilisation conforme à l'une des revendications précédentes, dans lequel l'ensemble raccord-auristatine est un fragment vcMMAE : 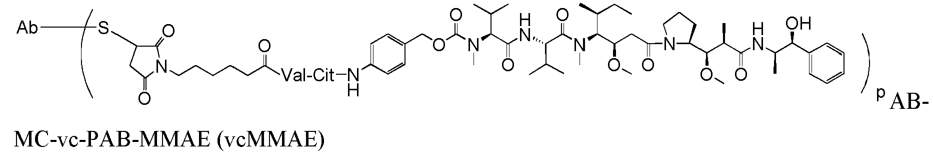 formule dans laquelle l'indice p est un nombre valant de 3 à 5 et de préférence 4, S représente un résidu de type sulfhydryle de l'anticorps anti-FT, et Ab représente l'anticorps anti-FT.

9. Conjugué anti-FT-AMC pour utilisation conforme à la reven-dication 2 ou 8, dans lequel l'indice p vaut en moyenne 4.

10. Conjugué anti-FT-AMC pour utilisation conforme à l'une des revendications précédentes, étant entendu que le conjugué anti-FT-AMC est administré le premier jour, le huitième jour et le quinzième jour du cycle de 28 jours.

11. Conjugué anti-FT-AMC pour utilisation conforme à l'une des revendications précédentes, étant entendu que la dose de conjugué anti-FT-AMC de 0,8 à 2,4 mg par kilogramme de poids corporel du sujet est une dose de 0,9 mg/kg, ou une dose de 1,0 mg/kg, ou une dose de 1,1 mg/kg, ou une dose de 1,2 mg/kg, ou une dose de 1,3 mg/kg, ou une dose de 1,4 mg/kg, ou une dose de 1,5 mg/kg, ou une dose de 1,6 mg/kg, ou une dose de 1,7 mg/kg, ou une dose de 1,8 mg/kg, ou une dose de 1,9 mg/kg, ou une dose de 2,0 mg/kg, ou une dose de 2,1 mg/kg, ou une dose de 2,2 mg/kg, ou une dose de 2,3 mg/kg.

12. Conjugué anti-FT-AMC pour utilisation conforme à l'une des revendications précédentes, étant entendu que le nombre de cycles de 28 jours vaut de 2 à 20, et que de préférence, le nombre de cycles vaut de 2 à 15, par exemple de 2 à 12, soit 2 cycles, 3 cycles, 4 cycles, 5 cycles, 6 cycles, 7 cycles, 8 cycles, 9 cycles, 10 cycles, 11 cycles, ou 12 cycles.

13. Conjugué anti-FT-AMC pour utilisation conforme à l'une des revendications précédentes, étant entendu que le conjugué anti-FT-AMC est administré pendant au moins quatre cycles de traitement de 28 jours, au cours desquels cycles le conjugué anti-FT-AMC, dans chaque cycle de traitement, est administré une fois par semaine, en une dose de 0,9 mg par kilogramme de poids corporel, pendant trois semaines consécutives, suivies d'une semaine de relâche sans adminis-tration de ce conjugué d'anticorps et de médicament.

14. Conjugué anti-FT-AMC pour utilisation conforme à l'une des revendications précédentes, étant entendu que le conjugué anti-FT-AMC est administré, en une dose de 0,9 mg par kilogramme de poids corporel, pendant au moins cinq cycles de traitement de 28 jours, soit 5, 6, 7, 8, 9, 10, 11 ou 12 cycles de traitement, au cours desquels cycles le conjugué anti-FT-AMC est administré une fois par semaine pendant trois semaines consécutives, suivies d'une semaine de relâche.

15. Conjugué anti-FT-AMC pour utilisation conforme à l'une des revendications précédentes, étant entendu que le conjugué anti-FT-AMC est administré en une dose de 0,9 mg par kilogramme de poids corporel, et que le cancer solide est l'un des suivants :
a) un cancer de la tête et du cou,
b) un cancer du poumon non à petites cellules,
c) un cancer du col de l'utérus,
d) ou un cancer des ovaires.

16. Conjugué anti-FT-AMC pour utilisation conforme à l'une des revendications 1 à 12, étant entendu que le conjugué anti-FT-AMC est administré en une dose de 1,2 mg par kilogramme de poids corpo-rel, et que le cancer solide est un cancer des ovaires.

17. Conjugué anti-FT-AMC pour utilisation conforme à l'une des revendications 1 à 12 et 16, étant entendu que le conjugué anti-FT-AMC est administré, en une dose de 1,2 mg par kilogramme de poids corporel, pendant au moins quatre cycles de traitement de 28 jours, soit 4, 5, 6, 7, 8, 9, 10, 11 ou 12 cycles de traitement, au cours desquels cycles le conjugué anti-FT-AMC est administré une fois par semaine pendant trois semaines consécutives, suivies d'une semaine de relâche.

18. Conjugué anti-FT-AMC pour utilisation conforme à l'une des revendications 1 à 12, étant entendu que le conjugué anti-FT-AMC est administré, en une dose de 1,5 mg par kilogramme de poids corpo-rel, pendant au moins quatre cycles de traitement de 28 jours, soit 4, 5, 6, 7, 8, 9, 10, 11 ou 12 cycles de traitement, au cours desquels cycles le conjugué anti-FT-AMC est administré une fois par semaine pendant trois semaines consécutives, suivies d'une semaine de relâche.

19. Conjugué anti-FT-AMC pour utilisation conforme à l'une des revendications précédentes, étant entendu que le traitement est suivi d'une thérapie d'entretien.

20. Conjugué anti-FT-AMC pour utilisation conforme à la revendication 19, étant entendu que la dose de conjugué anti-FT-AMC administrée pendant la thérapie d'entretien vaut d'environ 1 à environ 2,4 mg par kilogramme de poids corporel, telle une dose de 1,1 mg/kg, ou une dose de 1,2 mg/kg, ou une dose de 1,3 mg/kg, ou une dose de 1,4 mg/kg, ou une dose de 1,5 mg/kg, ou une dose de 1,6 mg/kg, ou une dose de 1,7 mg/kg, ou une dose de 1,8 mg/kg, ou une dose de 1,9 mg/kg, ou une dose de 2,0 mg/kg, ou une dose de 2,1 mg/kg, ou une dose de 2,2 mg/kg, ou une dose de 2,3 mg/kg.

21. Conjugué anti-FT-AMC pour utilisation conforme à la revendication 19 ou 20, étant entendu que la thérapie d'entretien est administrée selon un programme d'administration de 1 dose toutes les trois semaines, comme le premier jour d'un cycle de 21 jours.

22. Conjugué anti-FT-AMC pour utilisation conforme à la revendication 21, étant entendu que la thérapie d'entretien est adminis-trée selon des cycles de 21 jours et que le nombre de ces cycles vaut de 2 à 20, comme de 2 à 15 cycles, soit 2 cycles, 3 cycles, 4 cycles, 5 cycles, 6 cycles, 7 cycles, 8 cycles, 9 cycles, 10 cycles, 11 cycles, 12 cycles, 13 cycles, 14 cycles, ou 15 cycles.

23. Conjugué anti-FT-AMC pour utilisation conforme à l'une des revendications précédentes, étant entendu que le cancer solide est choisi dans l'ensemble comprenant des cancers du pancréas, de la tête et du cou, des ovaires, du col de l'utérus, de l'endomètre, de la vessie, de la prostate, de l'œsophage, ou du poumon.

24. Conjugué anti-FT-AMC pour utilisation conforme à la revendication 23, étant entendu que le cancer de la prostate est un cancer de la prostate résistant à la castration.

25. Conjugué anti-FT-AMC pour utilisation conforme à la revendication 23, étant entendu que le cancer du poumon est un cancer du poumon non à petites cellules.

26. Conjugué anti-FT-AMC pour utilisation conforme à l'une des revendications précédentes, étant entendu que le cancer solide exprime du FT.

27. Conjugué anti-FT-AMC pour utilisation conforme à l'une des revendications précédentes, étant entendu que le sujet présente un cancer solide exprimant du FT, réfractaire ou en rechute.

28. Conjugué anti-FT-AMC pour utilisation conforme à l'une des revendications précédentes, étant entendu que le conjugué anti-FT-AMC est administré en monothérapie.

29. Conjugué anti-FT-AMC pour utilisation conforme à l'une des revendications précédentes, étant entendu que le conjugué anti-FT-AMC est administré en tant que participant d'une thérapie combinée.

30. Conjugué anti-FT-AMC pour utilisation conforme à l'une des revendications précédentes, étant entendu que le conjugué anti-FT-AMC est compris dans une composition pharmaceutique.

31. Conjugué anti-FT-AMC pour utilisation conforme à la revendication 30, étant entendu que ladite composition pharmaceutique comprend en outre un véhicule pharmacologiquement admissible.

32. Conjugué anti-FT-AMC pour utilisation conforme à l'une des revendications précédentes, étant entendu que le conjugué anti-FT-AMC est administré par injection ou par perfusion, de préférence par perfusion intraveineuse.
